# EUROPEAN PATENT APPLICATION

(11) **EP 2 371 945 A1**
(43) Date of publication of application: **05.10.2011**
(21) Application number: 09829175.0
(22) Date of filing: 27.11.2009
(51) Int. Cl.: C12N 5/10, A61K 35/12, A61K 39/00, A61K 39/39, A61P 31/04, A61P 35/00, A61P 35/02, C12N 15/09

(54) **IMMUNOTHERAPEUTIC METHOD USING ALLO-CELLS WHICH CO-EXPRESS CD1d AND TARGET ANTIGEN**

(30) Priority: 28.11.2008 JP 2008305639
(71) Applicant: Riken, Wako-shi, Saitama 351-0198 (JP)
(72) Inventor: FUJII, Shin-ichiro, Yokohama-shi Kanagawa 230-0045 (JP); SHIMIZU, Kanako, Yokohama-shi Kanagawa 230-0045 (JP)
(74) Representative: Duckworth, Timothy John
(86) International application number: PCT/JP2009/070061
(87) International publication number: WO 2010/061930

(57) **Abstract**

The present invention provides a means to be used for a new immunotherapy of cancer or infection utilizing activation of dendritic cell (DC) by innate immunity, namely, a method of preparing a cell co-expressing a target antigen and CD1d and having an ability to activate immunity against the target antigen, comprising the following steps (a) and (b): (a) a step of transfecting an mRNA encoding the target antigen into a CD1d-expressing cell to give a cell co-expressing the target antigen and CDld; and (b) a step of treating the cell obtained in step (a) with a CD1d ligand in a culture medium.

## Description

### Technical Field

The present invention relates to a cell co-expressing target antigen and CD1d, which is used for a new immunotherapy of cancer or infection utilizing activation of dendritic cell (DC) by innate immunity, a cell co-expressing target antigen and CD1d, which has an ability to activate immunity against the target antigen, a method of producing them, an immunoinducer for a target antigen, and the like. More particularly, the present invention relates to a cell co-expressing target antigen and CD1d, which is used for an order-made antitumor or anti-infectious immunotherapy using an allo-cell transfected with an mRNA encoding an antigen characteristic of tumor or infection of individual, a cell co-expressing target antigen and CD1d and having an ability to activate immunity against the target antigen, a method of producing them, an immunoinducer for a target antigen and the like.

### Background Art

Heretofore, a direct effect on tumor and an adjuvant effect (indirect effect) via maturation of dendritic cell have been shown as antitumor effects of NKT cells. Based thereon, the inventions have established a method of inducing both an activation of NKT cell and a tumor-specific T cell immune response, including presenting an NKT cell ligand to autologous tumor cell expressing CD1d (patent document 1), and consider that the method is applicable to various antitumor immunotherapies of leukemia and the like.

In immunotherapy using NKT cells, an immunotherapy including administration of a dendritic cell presenting a tumor antigen has been performed for some time as a direct antitumor therapy for promoting activation of NKT cells, and directed to clinical application (non-patent documents 1-7).
On the other hand, in mouse models, it has been reported that administration of an NKT cell ligand affords induction of antigen-specific T cell immunity as an adjuvant effect, as evidenced by the result provided by the group of the present inventors. To use a method for simultaneous induction of the both, a tumor expressing CD1d was noted and the tumor cell was pulsed with an NKT cell ligand, α-GalCer, in vitro, whereby induction of activation of NK/NKT cells and T cells in vivo was confirmed. In other words, strong induction of innate immunity and acquired immunity could be confirmed. However, the problem in the studies heretofore was that application is difficult when the tumor cell is obtained only in a small amount from the patient.

Moreover, an attempt to induce an antigen-specific immunotherapy by transfecting an mRNA derived from a tumor antigen into a dendritic cell has already been established, and used for clinical applications (non-patent documents 8-15). However, the problem of this method is that mRNA transfection efficiency and expression level of tumor antigen in the dendritic cell are still weak, and an attempt has been made to improve the treatment effect by the concurrent use of an adjuvant.

### Prior Art Documents

### patent document

patent document 1: W02007/097370

### non-patent documents

non-patent document 1: Nat. Immunol. 3, 867-874 (2002).
non-patent document 2: J. Immunol. Meth. 272, 147-159 (2003).
non-patent document 3: J. Exp. Med. 198, 267-279 (2003).
non-patent document 4: J. Exp. Med. 199, 1607-1618 (2004).
non-patent document 5: J. Immunol. 167, 3114-3122 (2001).
non-patent document 6: Int. J. Cancer 109, 402-11 (2004).
non-patent document 7: J. Immunol. 171, 5140-5147 (2003).
non-patent document 8: J. Clin. Invest. 114, 1800-11 (2004).
non-patent document 9: J. Exp. Med. 184:465-472 (1996).
non-patent document 10: Nat. Med. 2:1122-1128 (1996).
non-patent document 11: Nat. Med. 6:1011-1017 (2000).
non-patent document 12: J. Clin. Invest. 109:409-417 (2002).
non-patent document 13: J. Immunol. 174:3798-3807 (2005).
non-patent document 14: Br. J. Cancer 93:749-756 (2005).
non-patent document 15: Cancer Gene Ther. 13:905-918 (2006).

### Summary Of The Invention

### Problems to be Solved by the Invention

When the treatment target is a solid tumor and the like, the difficulty of ensuring a sufficient amount of tumor cells used for the treatment is predicted. Moreover, when the treatment target is virus infection and the like, virus-infected autologous cells may not always be obtained in a sufficient amount and in a manner preferable for the treatment. The present inventors have pursued development of a method having higher function and higher versatility by improving the conventional method in which an NKT cell ligand is presented via aoutologous tumor cells or the like expressing CD1d. In addition, they have also pursued establishment of a highly safe method that suppresses, as much as possible, the possibility of side effects generally feared in gene therapy. Means of Solving the Problems

It has been considered desirable to use dendritic cells derived from a patient, in immunotherapy, since the autologous dendritic cell actually activates NKT cells and CD8⁺T cells in the body, and it has also been considered desirable to use the autologous tumor cells which the major histocompatibility complex (MHC) matches. However, the present inventors have unexpectedly found that allogeneic culture cells, which have been transfected with an mRNA encoding a target antigen and have been confirmed to have sufficient expression of the target antigen, can exhibit an effect equivalent to use of the autologous cell. This means that a cell usable for immunotherapy can be ensured with substantilally no limit, and this is an epoch-making achievement for the prevalence of the treatment. The present inventors have made further studies and developed a "cell kit" for immunotherapy, using an NKT cell ligand, mRNA of a tumor or virus and an allogeneic antigen presenting cell, which resulted in the completion of the present invention.

Accordingly, the present invention provides the following.
[1] A method of preparing a cell co-expressing a target antigen and CD1d and having an ability to activate immunity against the target antigen, comprising the following steps (a) and (b):
   (a) a step of obtaining a cell co-expressing the target antigen and CD1d by transfecting a CD1d-expressing cell with an mRNA encoding the target antigen; and
   (b) a step of treating the cell obtained in step (a) with a CD1d ligand in a culture medium.
[2] The method of [1], wherein the target antigen is a tumor antigen or a pathogenic antigen.
[3] The method of [1], wherein the ability to activate immunity is against a tumor cell, virus or virus-infected cell.
[4] The method of [3], wherein the tumor cell is a solid tumor cell or a tumor cell in a hematopoietic tissue.
[5] A cell having an ability to activate immunity against a target antigen, which is obtained by the method of any of [1] to [4].
[6] The cell of [5], wherein the target antigen is a tumor antigen or pathogenic antigen.
[7] The cell of [5], wherein the ability to activate immunity is against a tumor cell, virus or virus-infected cell.
[8] An immunoinducer to a target antigen, comprising the cell of [5].
[9] A composition comprising the cell of [5] and an adjuvant.
[10] A pharmaceutical agent comprising the cell of [5].
[11] The pharmaceutical agent of [10], which is a therapeutic agent for a solid tumor, a tumor in a hematopoietic tissue or an infection.
[12] A cell co-expressing a target antigen and CD1d, which is treated to co-express a target antigen and CD1d or to enhance expression of a target antigen and/or CD1d, and is one kind selected from the group consisting of the following (a) to (c) :
   (a) a cell naturally expressing CD1d, which is transfected with an mRNA encoding the target antigen;
   (b) a cell transformed with a vector expressing CD1d and transfected with an mRNA encoding the target antigen; and
   (c) a cell transfected with an mRNA encoding CD1d and transfected with an mRNA encoding the target antigen.
[13] A cell co-expressing a target antigen and CD1d, which is treated to co-express a target antigen and CD1d or to enhance expression of a target antigen and/or CD1d, and is one kind selected from the group consisting of the following (a) to (c):
   (a) an antigen presenting cell which expresses CD1d, and is transfected with an mRNA encoding the target antigen;
   (b) an antigen presenting cell which expresses CD1d, and is transformed with a vector expressing CD1d and transfected with an mRNA encoding the target antigen; and
   (c) an antigen presenting cell which expresses CD1d, and is transfected with an mRNA encoding CD1d and transfected with an mRNA encoding the target antigen.
[14] A kit comprising any of following (1) to (8):
   (1) a combination of (1-1) a CD1d-expressing cell, and (1-2) a construct for in vitro transcription of an mRNA encoding the target antigen;
   (2) a combination of (2-1) a CD1d-expressing cell, and (2-2) an mRNA encoding the target antigen;
   (3) a combination of (3-1) a construct for in vitro transcription of an mRNA encoding CD1d, (3-2) a construct for in vitro transcription of an mRNA encoding the target antigen, and (3-3) an object cell;
   (4) a combination of (4-1) a construct for in vitro transcription of an mRNA encoding CD1d and an mRNA encoding the target antigen, and (4-2) an object cell;
   (5) a combination of (5-1) a construct for in vitro transcription of an mRNA encoding CD1d, (5-2) an mRNA encoding the target antigen, and (5-3) an object cell;
   (6) a combination of (6-1) an mRNA encoding CD1d and the target antigen, (6-2) a construct for in vitro transcription of an mRNA encoding the target antigen, and (6-3) an object cell;
   (7) a combination of (7-1) an mRNA encoding CD1d and the target antigen, (7-2) an mRNA encoding the target antigen, and (7-3) an object cell; and
   (8) a combination of (8-1) an mRNA encoding CD1d and the target antigen, and (8-2) an object cell.
[15] The kit of [14], further comprising a CD1d ligand.
[16] The kit of [15], which is used for induction of immunity.
[17] The kit of [16], wherein the CD1d-expressing cell or the object cell is allogeneic to a target in need of immunity induction.
[18] The kit of [17], wherein the CD1d-expressing cell or the object cell is a fibroblast.
[19] A method of inducing immunity, comprising administering an effective amount of the cell of [5] to a test subject (human) or a test subject (excluding human) in need thereof, wherein the cell is allogeneic to the test subject.
[20] The method of [19], wherein the cell is a fibroblast.
[21] The method of [19], further comprising administering an adjuvant.
[22] Use of the cell of [5], for the preparation of an immunoinducer.
[23] Use of [22], further comprising use of an adjuvant.

### Effect of the Invention

According to the present invention, a CD1d ligand known as an NKT cell ligand is loaded into an allogeneic fibroblast, rather than its autologous dendritic cell or tumor cell, transfected with mRNA of the target antigen, and the cell thereof is administered to activate NKT cell and NK cell in vivo, whereby a sufficient treatment effect can be obtained. Furthermore, according to the present invention, an immunotherapy against a wide range of therapeutic target can be established by identifying mRNA in a target tumor cell or pathogen-infected cell once a small amount of these cells can be obtained. According to the present invention, moreover, a cell capable of activating immunity against the target antigen can be prepared by selecting an allo-cell capable of highly efficient expression of a protein from transfected mRNA and by transfecting the cell with mRNA alone. In this case, the immunotherapy is not dealt with as a gene therapy by not using a virus vector, therefore, this immunotherapy is not associated with a fear of side effects caused by modification of the cell genome.

### Brief Description of the Drawings

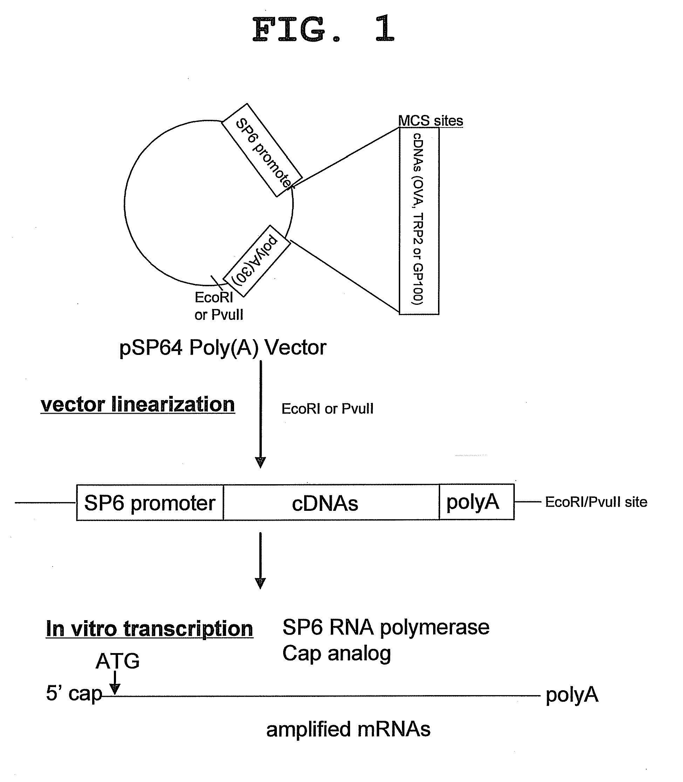

Fig. 1 is a schematic diagram showing the process of in vitro preparation of mRNA for the expression of a protein in a cell, from a full-length cDNA encoding the protein cloned into a vector.

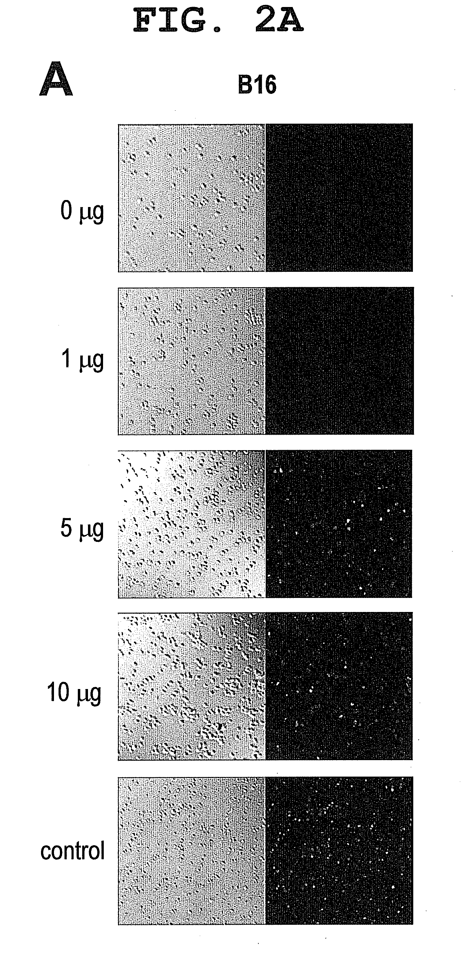

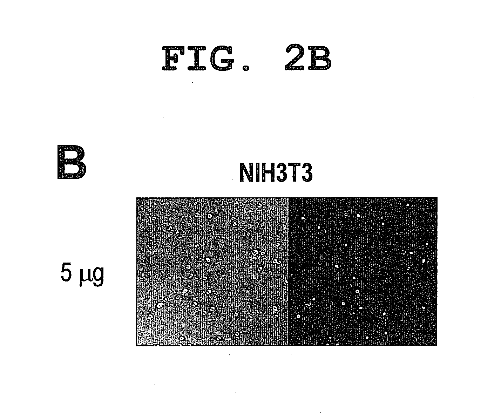

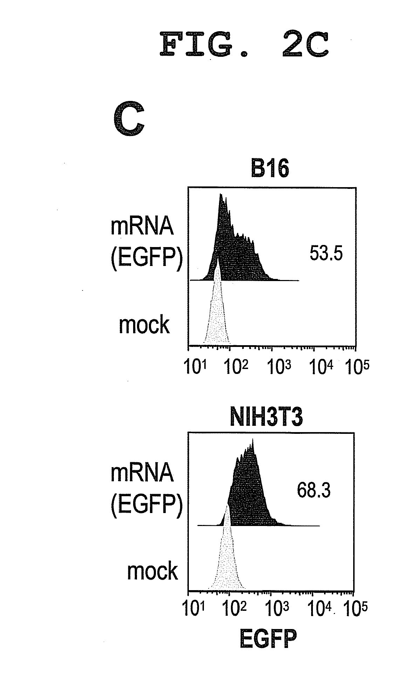

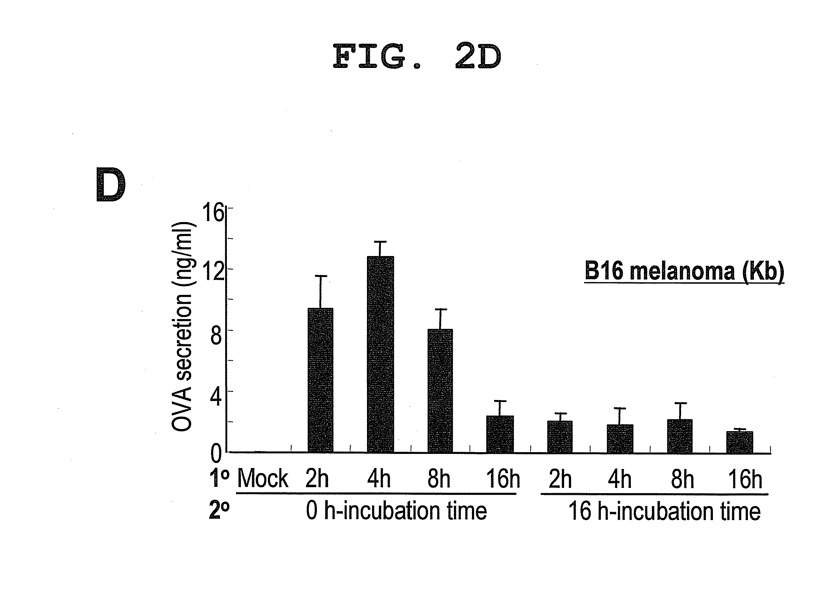

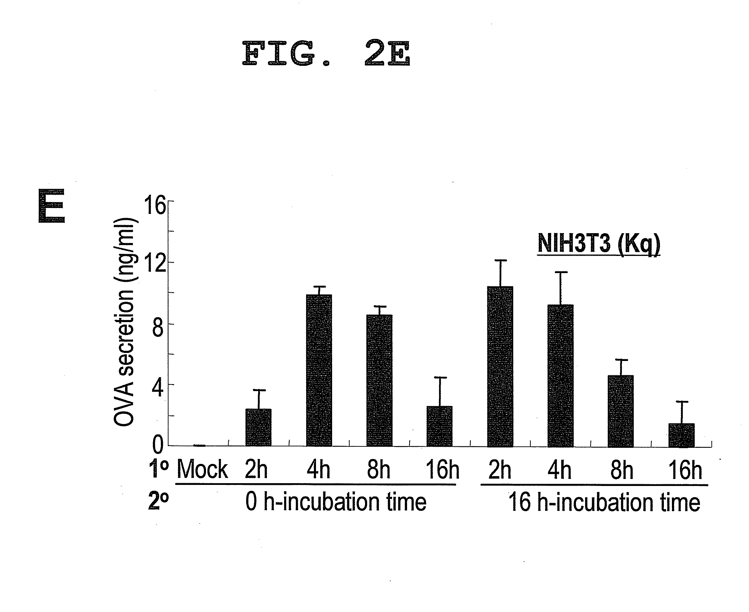

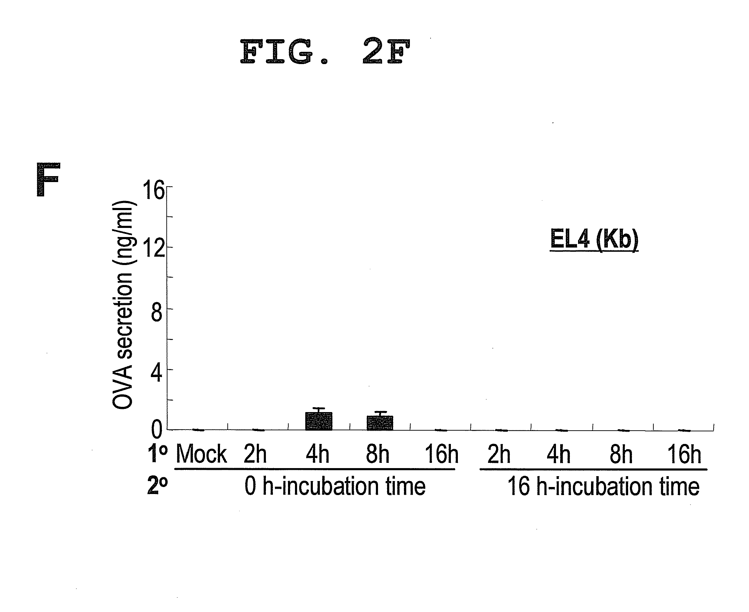

In Fig. 2, A shows confocal laser scanning microscopic images of GFP expression level of B16 melanoma cell transfected with EGFP mRNA in an amount shown on the left side of each image, B shows confocal laser scanning microscopic images of GFP expression level of NIH3T3 cell transfected with 5 µg of EGFP mRNA, C shows the results of FACS analysis of EGFP expression level of B16 (upper) or NIH3T3 (lower) cell transfected with 5 µg of EGFP mRNA, wherein 53.5 and 68.3 in the graphs respectively show the percentages of GFP positive cells, D shows the results of OVA expression levels as determined by ELISA of B16 melanoma cell after incubation for the time shown by 1° on the horizontal axis, transfection with 5 µg of OVA mRNA, and lapse of the time shown by 2°, E shows the results of OVA expression levels as determined by ELISA of NIH3T3 cell after incubation for the time shown by 1° on the horizontal axis, transfection with 5 µg of OVA mRNA, and lapse of the time shown by 2°, and F shows the results of OVA expression levels as determined by ELISA of EL4 cell after incubation for the time shown by 1° on the horizontal axis, transfection with 5 µg of OVA mRNA, and lapse of the time shown by 2°.

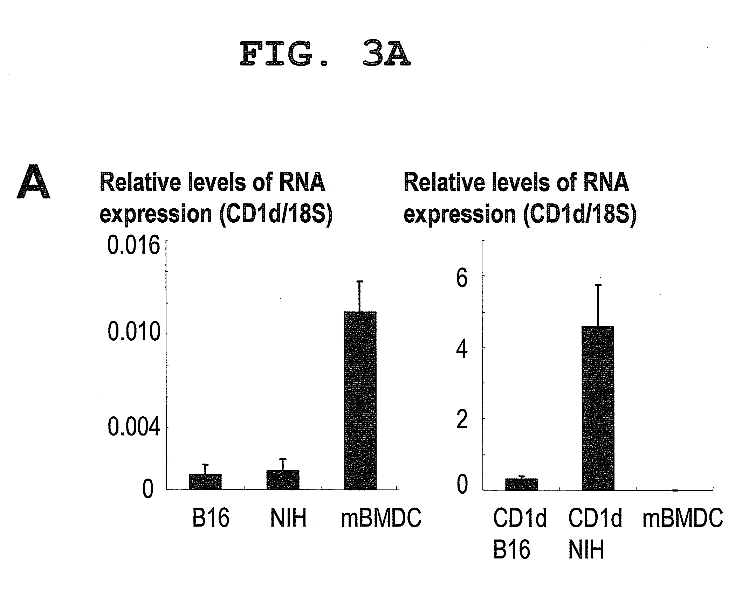

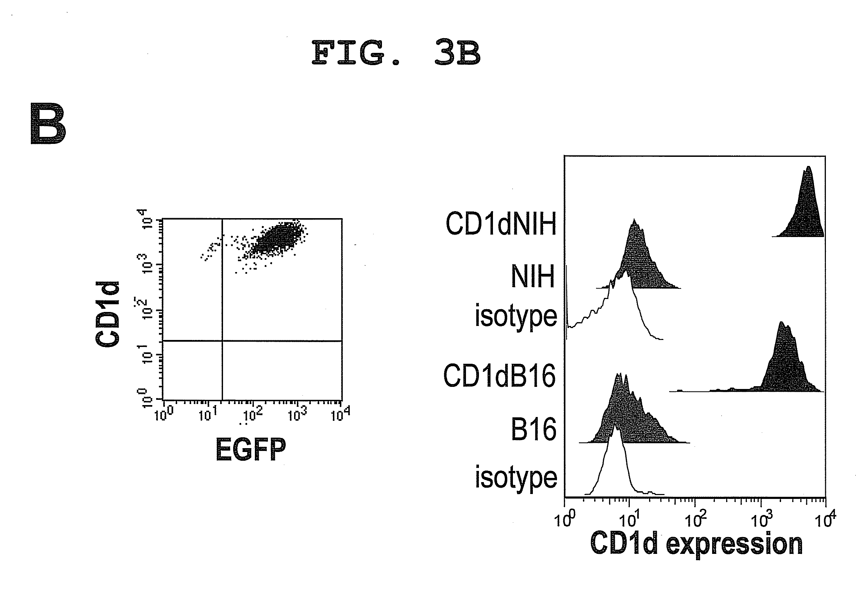

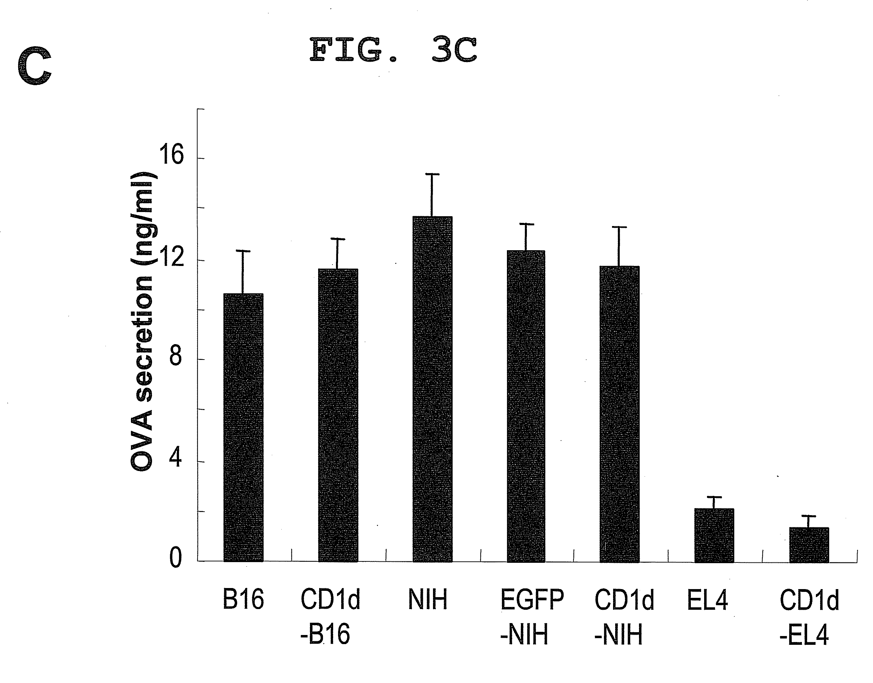

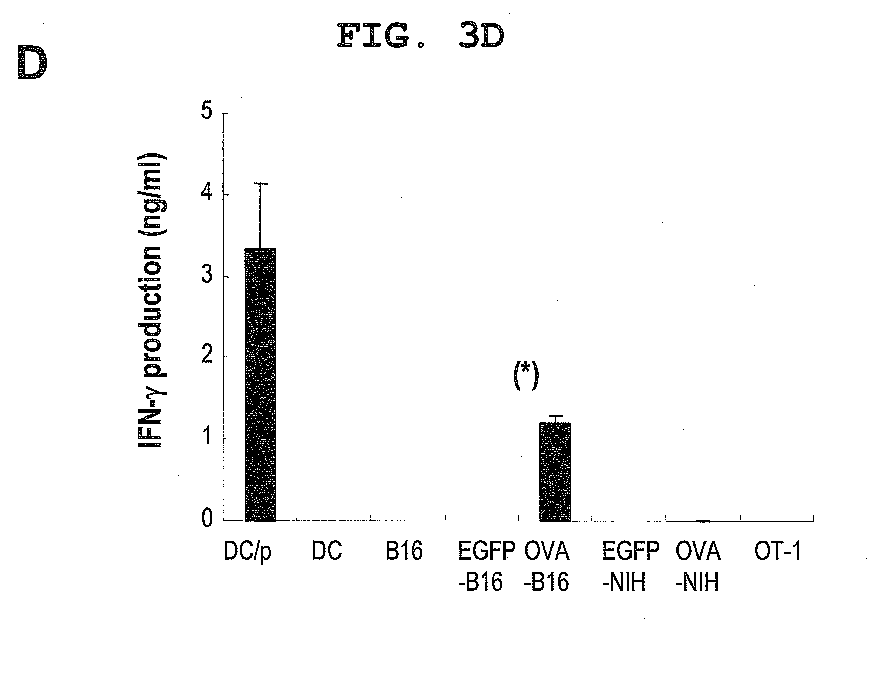

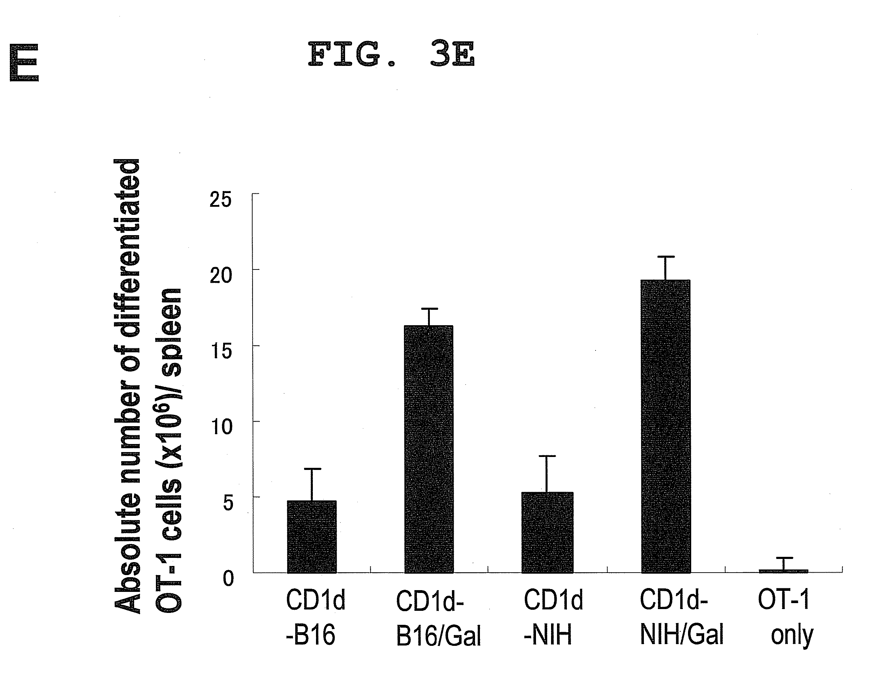

In Fig. 3, A shows the CD1d-expression level of each cell shown on the horizontal axis, which is quantified by real-time PCR and indicated by a relative value to rRNA, B shows CD1d and EGFP expression levels (Fig. left) and the results of FACS analysis of CD1d expression level of each cell shown on the vertical axis (Fig. right), C shows the results of OVA secretion amount of each cell shown on the horizontal axis, which was transfected with OVA mRNA, as measured by ELISA 4 hr later, D shows the results of IFN-γ secretion amount as measured by ELISA in the supernatant obtained by co-culture of CD8⁺T cell transformed with OVA (OT-I (OT-1 in the Figure) cell) and each cell shown on the horizontal axis, and E shows the results of OT-1 cell number of mouse spleen, which was obtained by giving OT-I cell, immunizing the mouse 24 hr later with an OVA mRNA transfectant loaded or not loaded with α-GalCer, and measuring the number 3 days later.

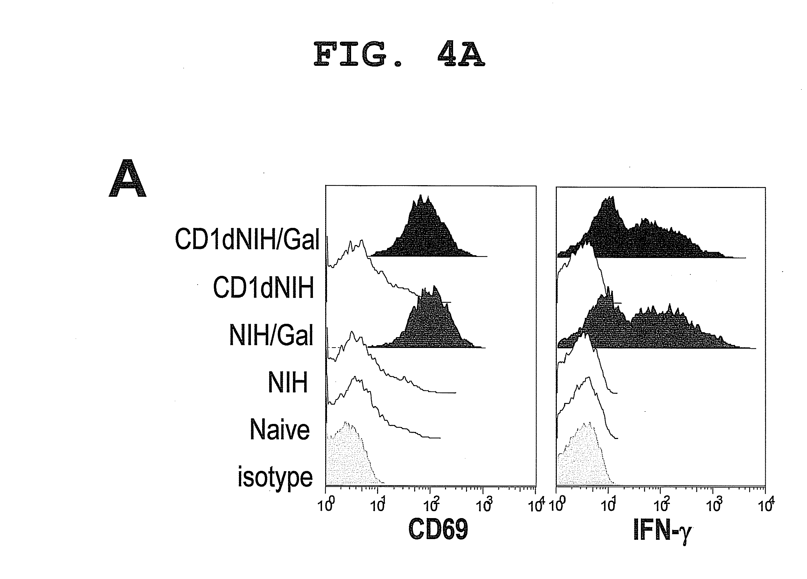

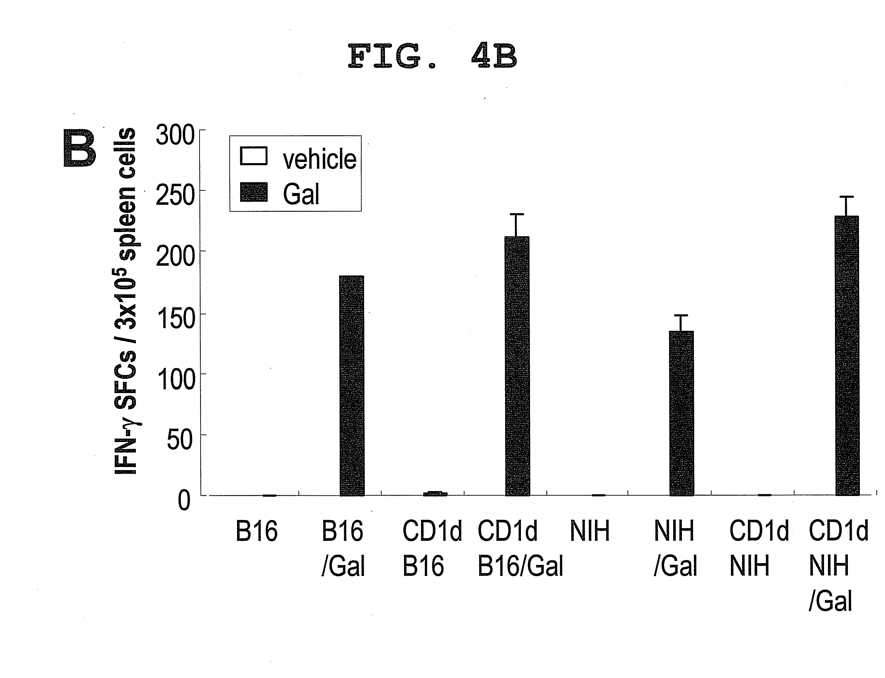

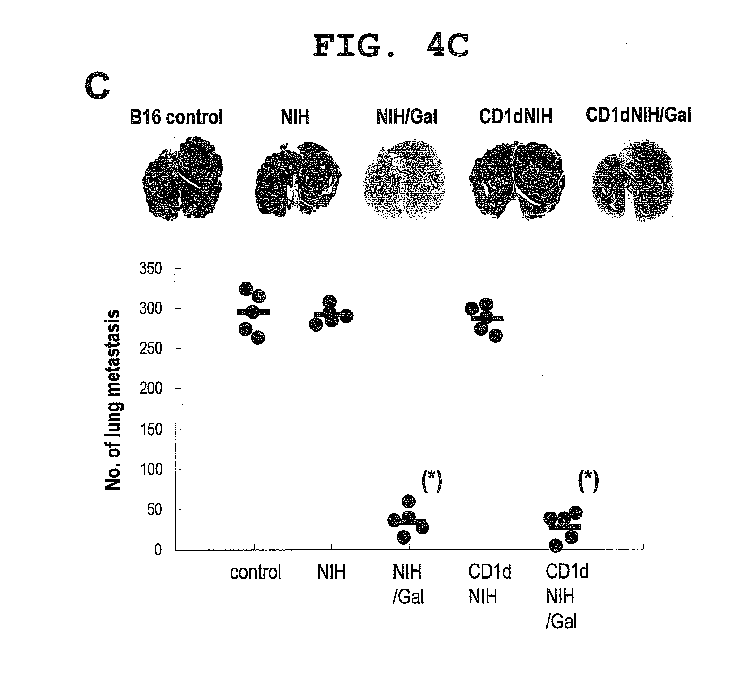

In Fig. 4, A shows the results of FACS analysis of the expression level of each protein shown on the horizontal axis in the mouse spleen cell immunized with each cell described on the vertical axis, B shows the level as measured by ELISPOT assay of IFN-γ produced by spleen cell of mouse immunized with each cell described on the horizontal axis, and C shows difference in antitumor immunity in lung metastasis models by the lung image and the number of lung metastasis.

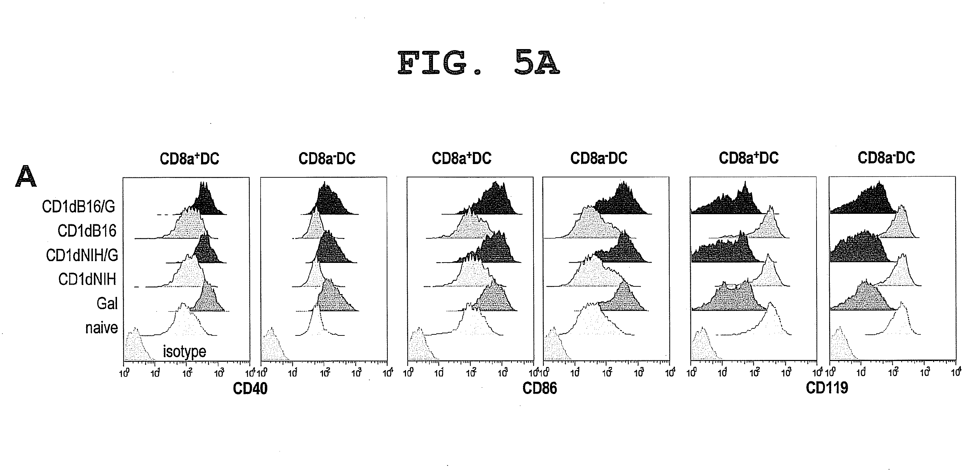

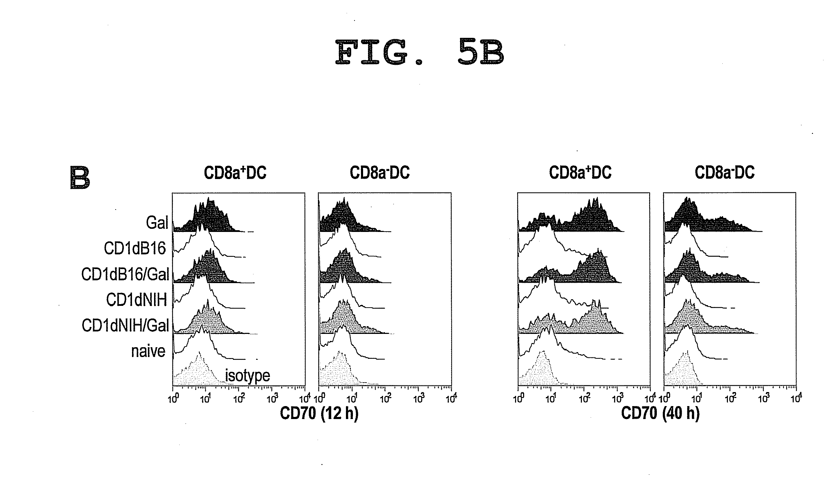

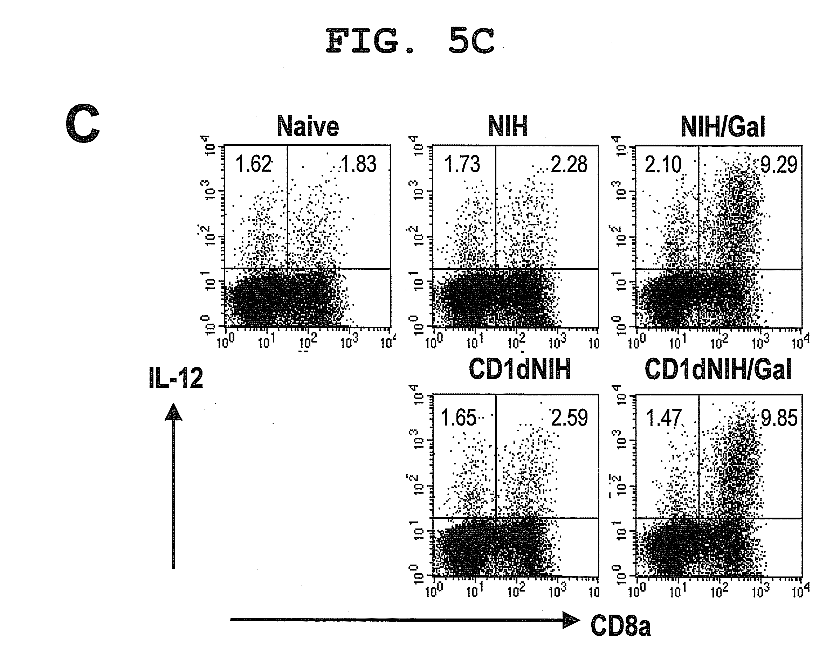

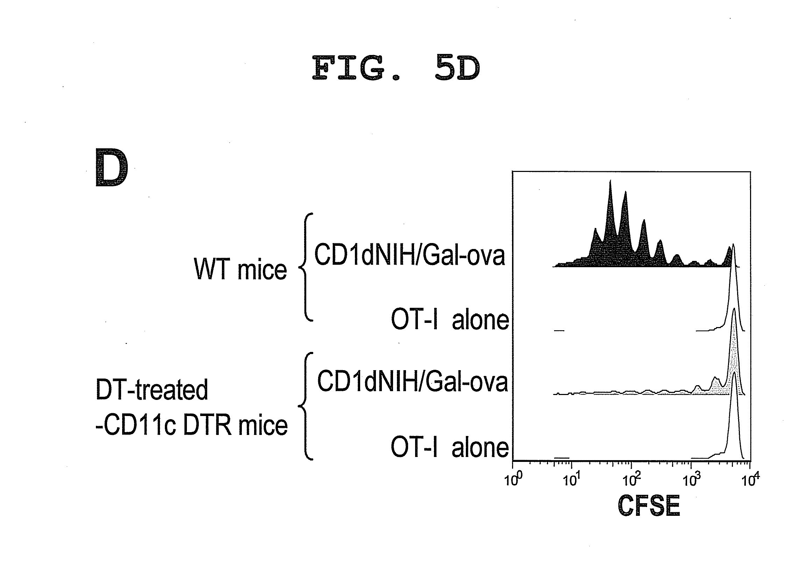

In Fig. 5, A shows the results of FACS analysis of the expression level of each protein shown on the horizontal axis in DC of mouse immunized with each cell described on the vertical axis and shown for CD8a⁺ and CD8a⁻ DC subsets, B shows CD70 expression levels of the mouse DC immunized with each cell described on the vertical axis, which was analyzed by FACS 12 hr and 40 hr after immunization and shown for CD8a⁺ and CD8a⁻ DC subsets, C shows CD8 and Ill expression levels of each cell by a flow cytometer, and D shows evaluation of OT-I cell growth in the mouse described in the vertical axis, which was administered with CFSE-labeled OT-I cell, with or without immunization with CD1d^{hi}-NIH3T3/Gal-ova (CD1dNIH/Gal-ova).

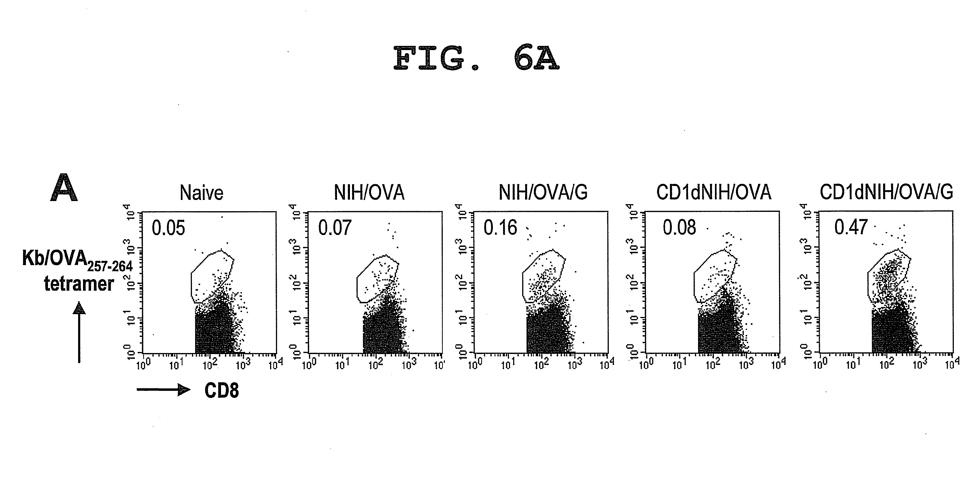

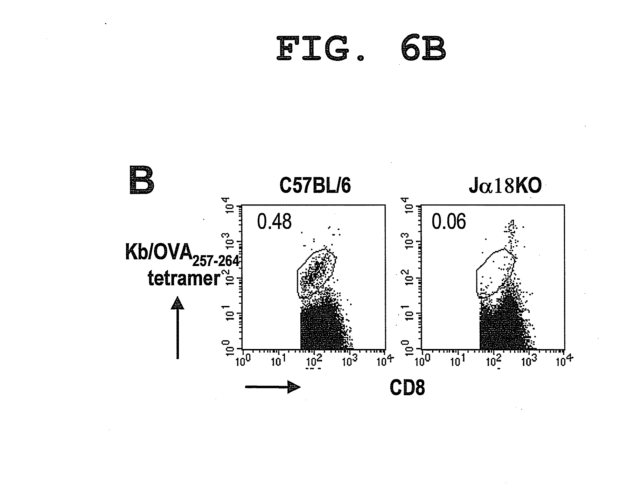

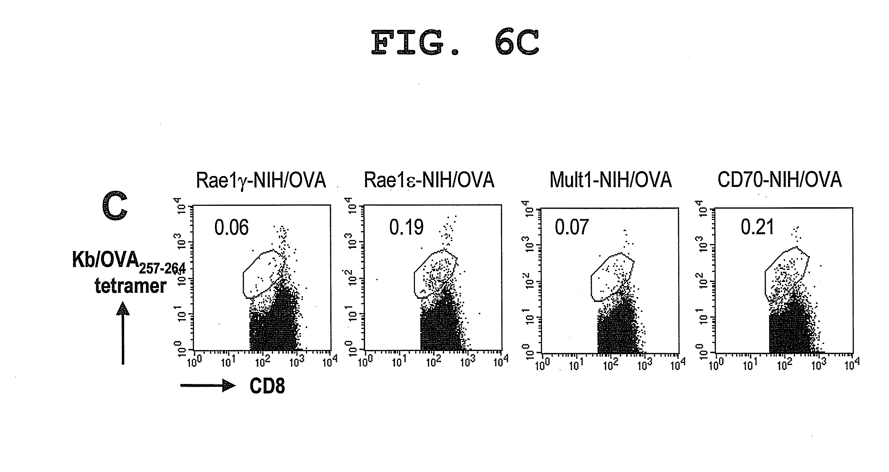

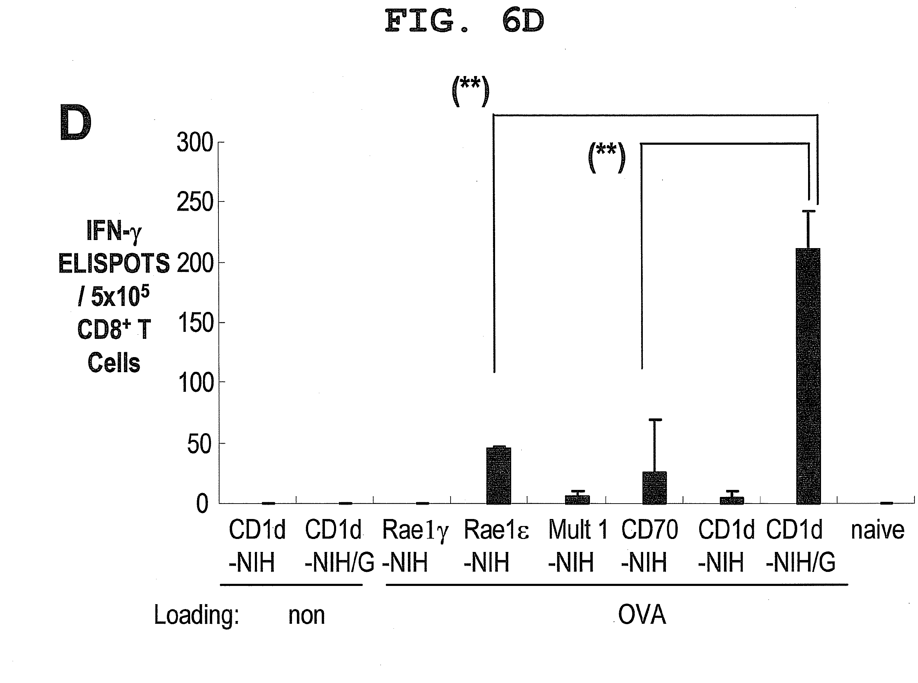

In Fig. 6, A shows the amounts of CD8 and OVA peptides on the spleen cell surface of the mouse immunized with the cell described in the upper part of each graph, B shows the amounts of CD8 and OVA peptides on the spleen cell surface of the mouse immunized with CD1d^{hi}-NIH3T3/Gal-ova cell and described in the upper part of each graph, C shows the amounts of CD8 and OVA peptides on the spleen cell surface of the mouse immunized with the cell described in the upper part of each graph, and D shows the quantification results of IFN-γ secreted by co-culture of CD8⁺ cell of the mouse immunized with the cell described on the horizontal axis and CDllc+ pulsed with OVA peptide.

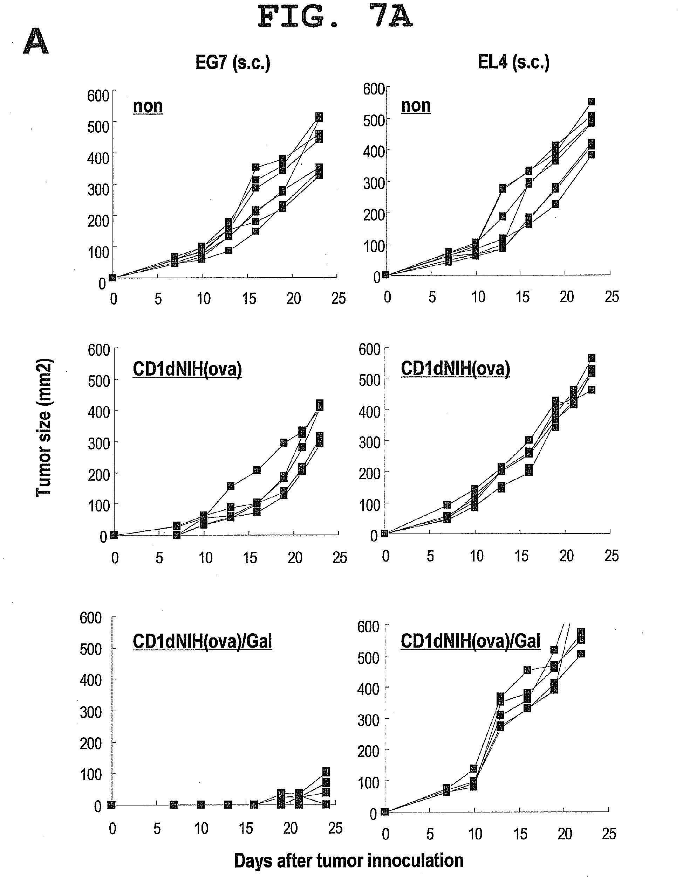

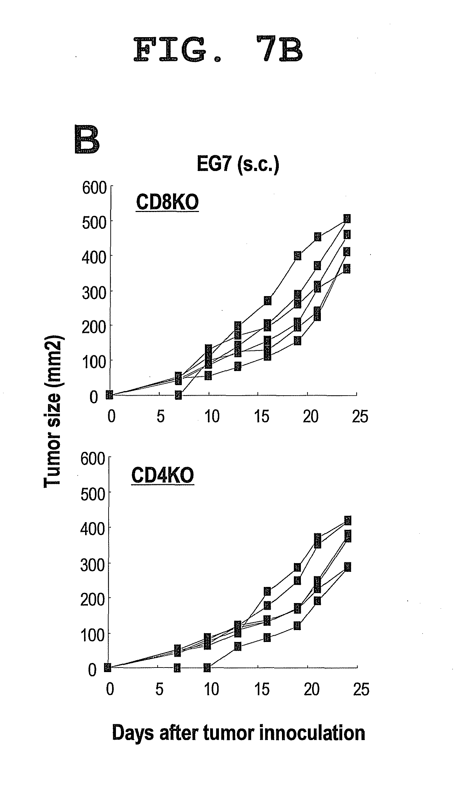

In Fig. 7, A shows the size of tumor in a mouse after immunization with the cell underlined in each graph, subcutaneous administration of EG7 (left) or EL4 (right) 2 weeks later, and lapse of the number of days shown on the horizontal axis, and B shows the size of tumor in a gene knockout mouse underlined in each graph after immunization with CD1d^{hi}-NIH3T3/Gal-ova, subcutaneous administration of EG7 2 weeks later, and lapse of the number of days shown on the horizontal axis.

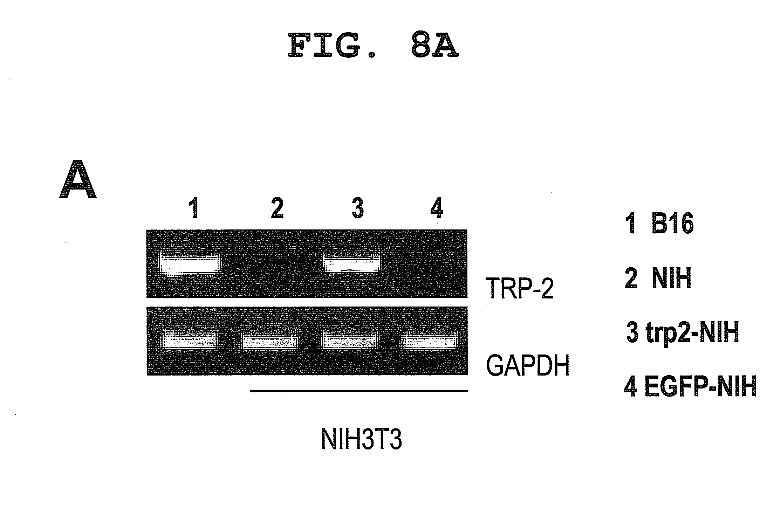

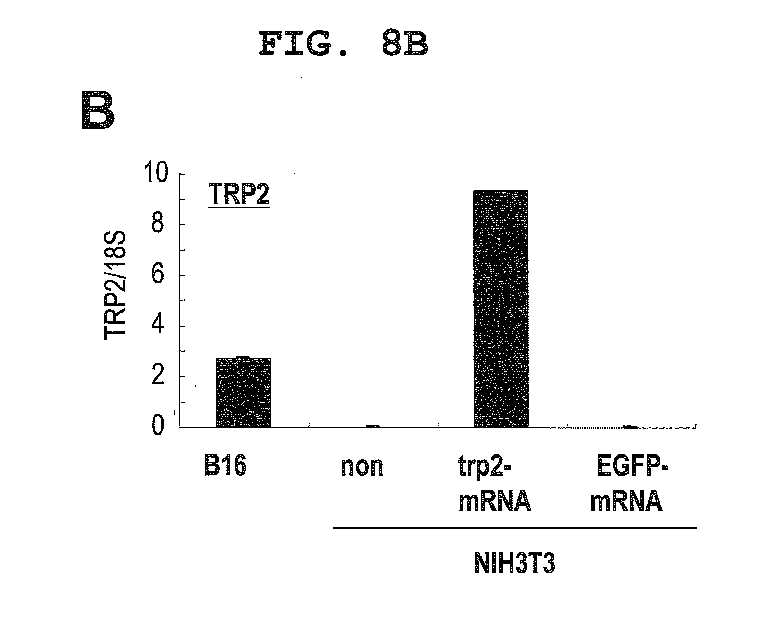

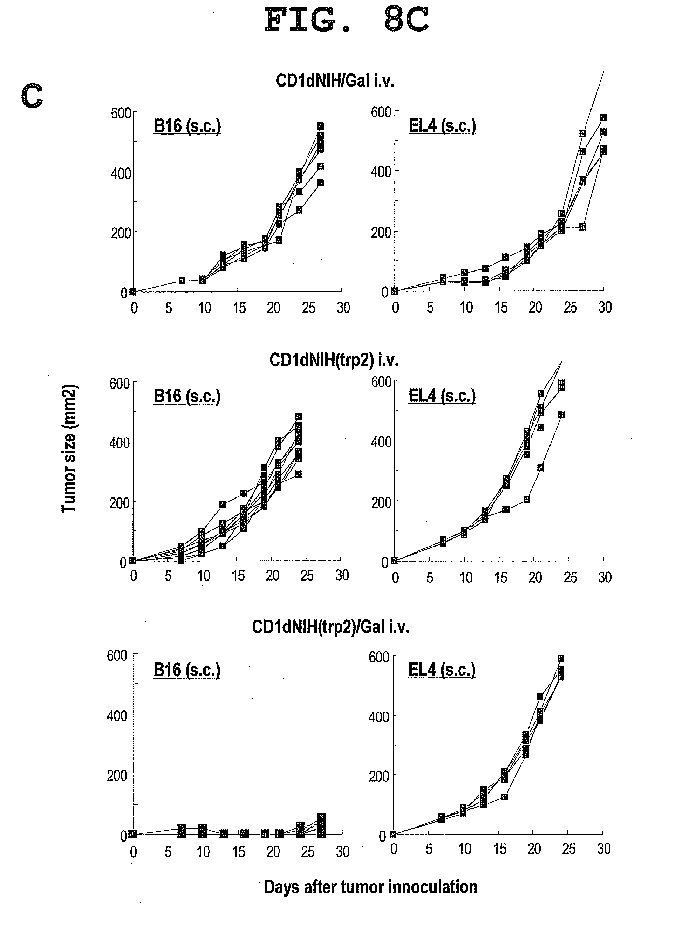

In Fig. 8, A shows the expression level of trp2 in a cell shown by each lane number as measured by RT-PCR, B shows the expression level of trp2 in each cell shown on the horizontal axis as quantified by real-time PCR, C shows the size of tumor in a mouse after immunization with a pair of the cells described in the upper right and upper left of each graph, subcutaneous administration of the cell underlined in each graph 2 weeks later, and lapse of the number of days shown on the horizontal axis.

### Description of Embodiments

### (1. Cell)

The present invention provides a cell co-expressing a target antigen and CD1d, which is loaded with a CD1d ligand, particularly a cell co-expressing a target antigen and CD1d, which is obtained by transfecting an mRNA encoding the target antigen into a CD1d-expressing cell. Such co-expressing cell loaded with a CD1d ligand has an ability to activate immunity against the target antigen. The cell of the present invention is characterized in that it is a cell derived from another individual of the same race to an individual to be immunized with the cell, that is a cell allogeneic to the subject of administration (allo-cell). In the following, the cell co-expressing a target antigen and CD1d, which is provided by the present invention, is also referred to as the allo-cell of the present invention.

CD1d ligand refers to a substance capable of activating NKT cells, presented on a CD1d-expressing antigen presenting cell (APC). Examples of "CD1d ligand" include α-GalCer (α-galactosylceramide), α-C-GalCer (α-C-galactosylceramide), iGB3 (isoglobotrihexosylceramide), GD3 (ganglioside 3), GSL-1 (α-linked glucuronic acid) and GSL-1'SA (galacturonic acid), with preference given to α-GalCer and α-C-GalCer. The allo-cell of the present invention presents CD1d ligand on its cell surface via CD1d, and can activate NKT cells.

Moreover, when an mRNA encoding the target antigen is transfected, the allo-cell of the present invention can highly express a protein encoded therewith. Examples of such cell include a cell that affords EGFP positive cells in a proportion of preferably not less than 50%, more preferably not less than 60%, of the whole cells; when 5 µg of mRNA encoding EGFP protein is transfected into the cells (2×10⁵ cells) by using a TransMessenger transfection kit (Qiagen) according to the protocol thereof and identified by FACS analysis 4 hr later. A cell in which transfection efficiency of target antigen mRNA and expression efficiency of its protein are high level may also be used even if transfection efficiency of EGFP mRNA and expression efficiency of EGFP protein are low level. When the expression efficiency of the protein encoded by mRNA to be transfected depends on the culture conditions and the like, optimal transfection conditions are determined by experiment, based on which the cell of the present invention can be produced.

The present invention also provides a novel cell from among predetermined cells that can be loaded with CD1d ligand. The present inventors have found for the first time that an allo-cell loaded with a CD1d ligand, which is a cell transfected with mRNA encoding the target antigen and co-expressing a target antigen and CD1d, is highly useful for immunotherapy. Therefore, the cell co-expressing a target antigen and CD1d produced in this way may be novel.

From among the allo-cells of the present invention, the cell loaded with CD1d ligand is referred to as "loaded cell of the present invention" (which means a cell that may have an ability to activate immunity against the target antigen) as necessary. Furthermore, from among the allo-cells of the present invention, the cell which is not loaded with a CD1d ligand is referred to as "unloaded cell of the present invention" (which means a cell that can acquire an ability to activate immunity against the target antigen after being loaded with a CD1d ligand, but do not have an ability to activate immunity against the target antigen since it is not loaded with CD1d ligand) as necessary.

The allo-cell of the present invention may be isolated and/or purified. Cell isolation and purification can be performed by a method known per se.

The allo-cell of the present invention may also be derived from any animal species. Examples of such animal species include mammals such as human, monkey, chimpanzee, dog, cat, horse, bovine, swine, sheep, goat, mouse, rat, guinea pig, hamster, rabbit and the like, with preference given to a cell derived from human from the aspect of clinical application.

Furthermore, the allo-cell of the present invention may be a cell type derived from any tissue. Examples of such tissue include stomach, small intestine (e.g., duodenum, jejunum, ileum, colon), large intestine, rectum, lung, pancreas, kidney, liver, thymus, spleen, thyroid gland, adrenal gland, prostate, ovary, uterus, bone marrow, skin, and peripheral blood. The allo-cell of the present invention may also be a particular cell type in the above-mentioned tissues or a cell type in a tissue other than the above-mentioned tissues. Examples of such cell type include epithelial cell, endothelial cell, epidermal cell, interstitial cell, fibroblast, adipocyte, mammary cell, mesangial cell, pancreatic P cells, nerve cell, glial cell, immune cell (e.g., T cell, B cell, NK cell, NKT cell, macrophage, mast cell, neutrophil, basophil, eosinophils, monocyte), and precursor cells and stem cells of these cells.

Furthermore, the allo-cell of the present invention may be a cell obtained from an animal (e.g., primary cultured cell) or a cell line. The cell line may be an existing cell line or a newly prepared cell line. The cell line can be prepared by a method known per se.

More importantly, the allo-cell of the present invention may be a cell that expresses both a target antigen and CD1d.

A target antigen is an antigen expressed on an abnormal cell or pathogen, and is not particularly limited as long as intracorporeal disappearance of the abnormal cell or pathogen, or a decrease in the amount of the abnormal cell or pathogen is expected by an immune reaction against the target antigen. Examples of the target antigen include tumor antigen and pathogenic antigen. The cell of the present invention can express one or more target antigens to the same target.

The tumor antigen may be an antigen of a solid tumor including epithelial and nonepithelial tumors, or a tumor in a hematopoietic tissue. While the solid tumor antigen is not particularly limited, for example, MART-1/Melan-A, Mage-1, Mage-3, gp100, tyrosinase, tyrosinase-related protein 2 (trp2), CEA, PSA, CA-125, erb-2, Muc-1, Muc-2, TAG-72, AES, FBP, C-lectin, NY-ESO-1, galectin-4/NY-CO-27, Pec60, HER-2/erbB-2/neu, telomerase, G250, Hspl05, point mutated ras oncogene, point mutated p53 oncogene and carcinoembryonic antigen can be mentioned (e.g., JP-A-2005-139118, JP-A-2004-147649, JP-A-2002-112780, JP-A-2004-222726). While the antigen of tumor in a hematopoietic tissue (e.g., leukemia) is not particularly limited, for example, proteinase 3, WT-1, hTERT, RAME, PML/RAR-a, DEK/CAN, cyclophilin B, TEL-MAL1, BCR-ABL, OFA-iLRP, Survivin, idiotype, Sperm protein 17, SPAN-Xb, CT-27 and MUC1 can be mentioned.

The pathogenic antigen may be a pathogenic virus antigen, a pathogenic microorganism antigen, or a pathogenic protozoan antigen. While a pathogenic virus antigen is not particularly limited, for example, antigens of viruses such as human immunodeficiency virus (HIV), hepatitis virus (e.g., type A, type B, type C, type D and type E hepatitis virus), influenza virus, simple herpes virus, West Nile fever virus, human papillomavirus, horse encephalitis virus, human T-cell leukemia virus (e.g., HTLV-I) and the like can be mentioned. Specifically, for example, GP-120, p17, GP-160 (HIV); NP, HA (influenza viruses); HBs Ag, HBV envelope protein, core protein, polymerase protein, NS3, NS5 (hepatitis viruses); HSVdD (simple herpes virus); EBNA1, 2, 3A, 3B and 3C, LMP1 and 2, BZLF1, BMLF1, BMRF1, BHRF1 (EB viruses); Tax (HTLV-I); SARS-CoV spike protein (SARS virus); CMV pp5, IE-1 (CMVs); E6, E7 proteins (HPVs) can be mentioned (e.g., JP-A-2004-222726). Examples of the pathogenic microorganism antigen include antigens expressed in pathogenic bacterium (e.g., chlamydiae, mycobacteria, Legionella) or pathogenic yeast (e.g., aspergillus, Candida). Examples of the pathogenic protozoan antigen include antigens expressed in malaria or schistosome.

CD1d is known to be a major histocompatibility complex (MHC)-like molecule that presents glycolipid rather than peptide. CD1d is also expressed in antigen presenting cell (e.g., dendritic cell), epithelial cells in tissues in the intestine, liver and the like, some tumor cells (e.g., solid tumor cell, leukemia cell) and virus-infected cells.

The allo-cell of the present invention may also be non-transfectant or transfectant. When used in the present specification, the transfection refers to an artificial transgenic operation, and the transfectant means a cell produced by such artificial operation. Therefore, a cell produced by a non-artificial operation is treated as one not falling under a transfectant in the present specification. To be more precise, when the cell of the present invention is a transfectant, the allo-cell of the present invention can be produced by using CD1d-expressing cell as a host cell and by transfecting the cell with mRNA encoding the target antigen (detail mentioned below). The host cell for transfection may be any cell expressing CD1d, and can be, for example, a cell naturally expressing CD1d or a cell prepared to express CD1d by an artificial operation.

The allo-cell of the present invention may also be a cell derived from an antigen presenting cell (APC) or non-APC, each being naturally-occurring. Examples of the naturally-occurring APC include dendritic cell, macrophage, B cell, Langerhans cell and activated T cell. Accordingly, the allo-cell of the present invention may be derived from a naturally-occurring APC or non-APC transfected with mRNA encoding the target antigen.

. The allo-cell of the present invention may also be a cell derived from an antigen presenting cell expressing CD1d (CDld-expressing APC) or non-CD1d-expressing APC, each being naturally-occurring. The CD1d-expressing APC refers to a cell capable of activating NKT cells, which has CD1d on the cell surface thereof. Examples of the CD1d-expressing APC include dendritic cell, macrophage and B cell. Accordingly, the allo-cell of the present invention may be derived from a naturally-occurring CD1d-expressing APC or non-CDld-expressing APC transfected with mRNA encoding the target antigen.

The allo-cell of the present invention may be derived from a naturally-occurring cell expressing a target antigen.

The loaded cell of the present invention is, as mentioned below, useful as a pharmaceutical agent, an immune activator and the like. The unloaded cell of the present invention is useful, for example, for the preparation of the loaded cell of the present invention.

### (2. Preparation and identification methods)

The present invention provides a method for preparing the allo-cell of the present invention.

In one embodiment, the preparation method of the present invention can be a method for preparing the unloaded cell of the present invention. The preparation method of the unloaded cell of the present invention may include treating a cell such that the target antigen and CD1d will be co-expressed in the object cell, or expression of the target antigen and/or CD1d will be enhanced in the cell co-expressing a target antigen and CD1d.

For example, when the preparation method of the present invention includes treatment of an object cell such that the target antigen and CD1d will be co-expressed in the cell, the object cell may be a cell that does not express both the target antigen and CD1d, or a CD1d-expressing cell.

In addition, when the preparation method of the present invention includes treatment of a cell such that the expression of the target antigen and/or CD1d will be enhanced in the cell co-expressing a target antigen and CD1d, the expression of the target antigen and/or CD1d can be enhanced to the degree that the treatment effect of the immunotherapy using the cell of the present invention is sufficiently increased.

The treatment in the preparation method of the present invention may be transformation or a operation to transfect mRNA of a target antigen. To be more precise, the preparation method of the present invention can include (a) transfecting an mRNA encoding a target antigen into a CD1d-expressing cell, (b) transfecting 1 or 2 molecular species of mRNA encoding a target antigen and CD1d into a cell or (c) transforming a cell with a CD1d-expressing vector, and then transfecting the cell with an mRNA encoding the target antigen. The transformation of the cell and mRNA transfection can be performed by a method known per se such as a lipofection method, a calcium phosphate precipitation method, an electroporation method and the like.
In the above-mentioned embodiment (b), the "1 molecular species of mRNA encoding a target antigen and CD1d" means both the target antigen and CD1d are encoded by one mRNA, and the "two molecular species of mRNA encoding a target antigen and CDld" means each of the target antigen and CD1d is encoded by a separate mRNA.
Here, the target antigen to be expressed in an object cell may be one or more kinds. That is, mRNA of the target antigen to be used for the preparation method of the allo-cell may be an mRNA derived from one kind of antigen or a mixture of mRNAs derived from plural kinds of antigens (there are plural kinds of mRNA encoding the antigen).

In the aforementioned preparation method, the CD1d-expressing cell in (a) may be a cell which expresses CD1d and does not express a target antigen. The cell in (b) and (c) can be a cell which does not express both of the target antigen and CD1d.

In another embodiment, the preparation method of the present invention may be a preparation method of the loaded cell of the present invention. The preparation method of the loaded cell of the present invention may include treating a cell co-expressing a target antigen and CD1d, for example the unloaded cell of the present invention, with a CD1d ligand in a culture medium. By such a treatment, the CD1d ligand is presented on the cell co-expressing a target antigen and CD1d, and the co-expressing cell can acquire an ability to activate immunity against the target antigen.

The culture medium can be prepared using, as a basal medium, a medium used for culturing animal cells. Examples of the basal medium include MEM medium, DMEM medium, αMEM medium, Ham's medium, RPMI1640 medium, Fischer's medium, and a mixed medium thereof. The culture medium can contain, for example, serum (e.g., FCS), serum replacement (e.g., knockout Serum Replacement (KSR)), fatty acid or lipid, amino acid, vitamin, growth factor, cytokine, antioxidant, 2-mercaptoethanol, pyruvic acid, buffering agent, inorganic salts and the like. Other culture conditions such as culture temperature, CO₂ concentration and the like can be set as appropriate. While the culture temperature is not particularly limited, for example, it is about 30 - 40°C, preferably about 37°C. The CO₂ concentration is, for example, about 1 - 10%, preferably about 5%. Other conditions such as the number of cells to be cultured, concentrations of various factors and the like can be appropriately set by methods known per se.

When the treated cell (e.g., transformant) is used as a cell co-expressing a target antigen and CD1d , the preparation method of the present invention may further include treating a cell such that the target antigen and CD1d will be co-expressed in the object cell or the expression of the target antigen and/or CD1d will be enhanced in the cell co-expressing a target antigen and CD1d, and obtaining the cell co-expressing a target antigen and CD1d. The methodology is the same as for the aforementioned preparation method of the present invention. The object cell is a cell derived from an individual of the same species (allogeneic cell) as an individual to which the cell of the present invention is administered.

### (3. Construct for in vitro transcription and mRNA)

The present invention provides a construct for in vitro transcription to produce an mRNA encoding a target antigen. Examples of the construct include a template construct transcribing only a target antigen mRNA, and a template construct transcribing a target antigen mRNA and mRNA of other useful factor. When plural kinds of target antigen mRNAs are used, respective mRNAs may be present in the same construct or separately present in different constructs. Examples of the construct for transcription in vitro to produce an mRNA encoding the target antigen include a template construct derived from a target antigen expression vector, and a template construct derived from a vector for co-expression of a target antigen and CD1d.

Similarly, a construct for in vitro transcription of an mRNA encoding CD1d is provided. Examples of the construct include a template construct for expression of only CD1d mRNA, and a template construct for expression of CD1d mRNA and mRNA of the other useful factor. Examples of the construct for in vitro transcription to produce an mRNA encoding CD1d include a template construct derived from a CD1d-expression vector, and a template construct derived from a vector for co-expression a target antigen and CD1d.

In the same manner, a construct for in vitro transcription of 1 or 2 molecular species of mRNA encoding a target antigen and CD1d is provided. Examples of the construct include a template construct for expression of both a target antigen mRNA and CD1d mRNA, and a combination of a template construct for expression of at least a target antigen mRNA and a template construct for expression of at least CD1d mRNA. Examples of the construct for in vitro transcription to produce of 1 or 2 molecular species mRNA encoding a target antigen and CD1d include a template construct derived from a vector for co-expression of a target antigen and CD1d, and a combination of a template construct derived from a target antigen-expression vector and a template construct derived from a CD1d-expression vector. When plural kinds of target antigen mRNAs are used, respective mRNAs may be present in the same construct or separately present in different constructs.

The present invention also provides a construct for co-expression of such mRNAs.

The co-expression construct of the present invention may contain a first polynucleotide encoding a target antigen and a second polynucleotide encoding CD1d. When plural kinds of target antigens are the targets, respective target antigens may be contained in the same polynucleotide or separately contained in different polynucleotides. The co-expression construct of the present invention may also contain a promoter operably linked to the above-mentioned first and the second polynucleotides. The operable linkage of the promoter means that the promoter is bound to the polynucleotide in such a manner as to permit expression of a factor encoded by the polynucleotide under regulation of the promoter.

To be more precise, the co-expression construct of the present invention may be a polycistronic mRNA expression construct. The polycistronic mRNA expression construct may contain a conjugate of a first polynucleotide (encoding one or more target antigens) and second polynucleotide, which enables expression of polycistronic mRNA of one or more target antigens and CD1d, and a promoter operably linked to the conjugate.

The co-expression construct of the present invention may also be a non-polycistronic mRNA expression vector. The non-polycistronic mRNA expression construct may contain a first polynucleotide and a first promoter operably linked to the polynucleotide, as well as a second polynucleotide and a second promoter operably linked to the polynucleotide. When plural kinds of target antigens are the targets, respective target antigens may be contained in the same polynucleotide or separately contained in different polynucleotides.

The promoter to be used for the construct for in vitro transcription is not particularly limited as long as it is operable in vitro and, for example, T7 promoter, SP6 promoter, T3 promoter and the like can be mentioned.

The construct for in vitro transcription preferably contains a transcription termination signal, i.e., terminator region, at the downstream of oligo(poly)nucleotide encoding a nucleic acid molecule. From the aspects of stability of synthesized mRNA and the like, moreover, it preferably contains a polyA sequence. Examples of the above-mentioned terminator sequence include SP6 terminator, T7 terminator, T3 terminator and the like. To amplify the co-expression construct itself, it may further contain a selection marker gene (gene imparting resistance to pharmaceutical agents (e.g., tetracycline, ampicillin, kanamycin, hygromycin, phosphinothricin), gene complementing auxotrophic mutation etc.) for selecting a transformed cell, which functions as a vector. For amplification, PCR may also be used.

When the construct for transcription in vitro is derived from a vector, the backbone thereof may be derived from, for example, a plasmid or a virus vector (e.g., vector derived from virus such as adenovirus, retrovirus, adeno-associated virus, herpesvirus, vaccinia virus, poxvirus, polio virus, sindbis virus, Sendai virus, lentivirus and the like).

The mRNA encoding the target antigen and the mRNA encoding CD1d, or the mRNA encoding target antigen and CD1d (hereinafter to be also referred to simply as the mRNA of the present invention) to be used in the present invention are prepared by a known method using the above-mentioned construct of the present invention and a commercially available in vitro transcription kit and the like. The template construct to be contained in the transcription reaction mixture may be circular or linear. When it is a circular DNA, it may be linearized by digestion with a restriction enzyme that recognizes the restriction enzyme site at an appropriate position. The template constructs to be used in the present invention are not particularly limited in the number of bases, and they may not have the same number of bases as long as the object protein can be synthesized. As long as it is a sequence homologous to the degree permitting synthesis of the object protein, moreover, each template construct may contain plural bases which have been deleted, substituted, inserted or added. From the aspects of stability, it desirably has a 5' cap structure.

The mRNA of the present invention is useful, like the below-mentioned agent of the present invention, for a pharmaceutical agent, the activation of immunocytes and preparation of the allo-cell of the present invention.

### (4. Agent and composition)

The present invention provides an agent (or composition) containing the allo-cell of the present invention, particularly the loaded cell of the present invention loaded with a CD1d ligand.

The animal species to which the agent of the present invention is administered may be the same as the animal species from which the allo-cell of the present invention is derived. Thus, the agent of the present invention can achieve alloimmunization in the immunization with the allo-cell of the present invention.

The agent of the present invention may contain, in addition to the loaded cell of the present invention, any carrier, for example, pharmaceutically acceptable carriers and/or adjuvant. Examples of the pharmaceutically acceptable carrier include, but are not limited to, diluents such as water, saline and the like. While the adjuvant is not particularly limited as long as it can enhance the antigenicity of the target antigen, for example, BCG, trehalose dimycolate (TDM), Merck65, AS-2, aluminum phosphate, aluminum hydroxide, keyhole limpet hemocyanin, dinitrophenol, dextran and TLR ligand (e.g., lipopolysaccharide (LPS), CpG) can be mentioned.

The agent of the present invention is useful, for example, as a pharmaceutical agent or reagent. To be more precise, the agent of the present invention is useful for the prophylaxis or treatment of neoplastic diseases or infections, or immunotherapy (e.g., activation of immunocytes such as NK/NKT cells, T cells and the like). Examples of the neoplastic diseases possibly prevented or treated by the agent of the present invention include tumors in the aforementioned tissues and cell types, such as solid tumor (e.g., epithelial tumor, non-epithelial tumor), and tumors in hematopoietic tissues. To be more precise, examples of the solid tumor possibly prevented or treated by the agent of the present invention include digestive organ cancer (e.g., gastric cancer, colon cancer, colorectal cancer, rectal cancer), lung cancer (e.g., small cell cancer, non-small cell cancer), pancreatic cancer, kidney cancer, liver cancer, thymus, spleen, thyroid cancer, adrenal gland cancer, prostate cancer, urinary bladder cancer, ovarian cancer, uterus cancer (e.g., endometrial carcinoma, cancer of the uterine cervix), bone cancer, skin cancer, sarcoma (e.g., Kaposi's sarcoma), melanoma, blastoma (e.g., neuroblastoma), adenocarcinoma, planocellular carcinoma, non-planocellular carcinoma, brain tumor, as well as recurrence and metastasis of these solid tumors. Examples of the tumor in the hematopoietic tissue possibly prevented or treated by the agent of the present invention include leukemia (e.g., acute myeloid leukemia (AML), chronic myelocytic leukemia (CML), acute lymphocytic leukemia (ALL), chronic lymphocytic leukemia (CLL), adult T cell leukemia (ATL), myelodysplastic syndrome (MDS)), lymphoma (e.g., T lymphoma, B lymphoma, Hodgkin's lymphoma), myeloma (multiple myeloma), as well as recurrence of these tumors. Examples of the infections possibly treated by the agent of the present invention include infections caused by the aforementioned pathogens.

The present inventors have also found at this time that the loaded cell of the present invention can have an ability to simultaneously induce activation of NK/NKT cells and T-cell immune response. It is known that MHC class I-non-expressing cell is a good target for NK/NKT cells, and MHC class I-expressing cell is a good target for T cells. Accordingly, the agent of the present invention is advantageous in that an effect on various target antigen-expressing cells can be expected.

While the dose of the agent of the present invention varies depending on the kind of the loaded cell of the present invention, expression levels of the target antigen and CD1d in the loaded cell of the present invention, administration mode, severity of disease, animal species of the subject of administration, and acceptability, body weight, age and the like of the subject of administration and it cannot be generalized, cells in a number that affords a desired immunoactivity can be appropriately administered. The agent (or composition) of the present invention can also be used as a vaccine, and can also be used as an agent for inducing immunity against a target antigen. When targetting plural kinds of target antigens (for example, when plural target antigen-derived mRNAs are transfected into an object cell etc.), an allo-cell presenting polyvalent antigen can be obtained, and such allo-cell can be used as a polyvalent vaccine, and as an agent for inducing immunity against plural target antigens.

Examples of the target antigen mRNA used for preparation of the agent (including vaccine) of the present invention include, but are not limited to, mRNA of melanocyte differentiation antigen and tyrosinase-relating protein 2 (trp2). The allo-cell of the present invention is prepared using a target antigen mRNA, and the loaded cell of the present invention is prepared by loading a CD1d ligand, and the obtained cell is used for immunization of an individual, whereby the growth of a cell characteristically containing the target antigen in the body can be specifically and markedly suppressed.

In the present invention, as a means for producing a CD1d-expressing cell, a CD1d-expression vector can be used. As the CD1d-expression vector, those similar to the construct for the in vitro transcription of an mRNA encoding CD1d as mentioned above can be utilized.

### (5. kit)

The above-mentioned various substances and/or cells can be formed as a kit as necessary.

To be more precise, the kit of the present invention is largely divided into a kit containing, as essential constituent components, a component for expressing a target antigen and a component for expressing CD1d (kit I), a kit further containing a CD1d ligand as an essential constituent component (kit II), and a kit further containing an expression measurement means as an essential constituent component (kit III).

The kit I of the present invention may contain, for example, any of (1) to (8) below:
(1) a combination of (1-1) a CD1d-expressing cell, and (1-2) a construct for in vitro transcription of an mRNA encoding the target antigen;
(2) a combination of (2-1) a CD1d-expressing cell, and (2-2) an mRNA encoding the target antigen;
(3) a combination of (3-1) a construct for in vitro transcription of an mRNA encoding CD1d, (3-2) a construct for in vitro transcription of an mRNA encoding the target antigen, and (3-3) an object cell;
(4) a combination of (4-1) a construct for in vitro transcription of an mRNA encoding CD1d and an mRNA encoding the target antigen, and (4-2) an object cell;
(5) a combination of (5-1) a construct for in vitro transcription of an mRNA encoding CD1d, (5-2) an mRNA encoding the target antigen, and (5-3) an object cell;
(6) a combination of (6-1) an mRNA encoding CD1d and the target antigen, (6-2) a construct for in vitro transcription of an mRNA encoding the target antigen, and (6-3) an object cell;
(7) a combination of (7-1) an mRNA encoding CD1d and the target antigen, (7-2) an mRNA encoding the target antigen, and (7-3) an object cell; and
(8) a combination of (8-1) an mRNA encoding CD1d and the target antigen, and (8-2) an object cell.

The kit II of the present invention contains, in addition to the respective constitution components of the above-mentioned kit I, a CD1d ligand as an essential constitution component. The CD1d ligand to be contained in kit II is similar to those mentioned above. Preferred are α-GalCer and α-C-GalCer.

The kit III of the present invention may contain a means capable of measuring the expression of a target antigen, and a means capable of measuring the expression of CD1d. Examples of the means capable of measuring the expression of a target antigen include antibody.

The promoter used for in vitro mRNA transcription, a construct to be the backbone and other factors for the kit of the present invention may be the same as those used for the construct of the present invention.

The CD1d-expressing cell to be contained in the kit of the present invention may be a cell that expresses CD1d and does not express a target antigen. The object cell to be contained in the kit of the present invention may be a cell that does not express CD1d and a target antigen, or a cell that expresses CD1d only at a low expression level and does not express a target antigen. The cell to be used in the present invention is characteristically a cell derived from another individual allogeneic to an individual to be immunized with the cell, that is, a cell allogeneic to subject of administration (allo-cell).

The kit of the present invention may further contain the aforementioned adjuvant.

The kit of the present invention may also contain a reagent that can confirm activation of an immunocytes. Examples of the reagent that can confirm activation of immunocytes include a reagent for the measurement of the number of one or more immunocytes selected from the group consisting of NK cell, NKT cell and T cell, and a reagent for the measurement of a substance specific to the activated immunocyte.

The reagent for the measurement of the number of immunocytes may contain, for example, specific antibodies against cell surface markers (e.g., Vα24, Vβ11) of NK cell, NKT cell and T cell, or nucleic acid probes capable of detecting transcription products encoding the markers or plural primers (e.g., primer pair(s)) capable of amplifying them.

The reagent for the measurement of a substance specific to the activated immunocyte may contain, for example, antibodies against substances (e.g., IFN-γ, perforin, granzyme B) specific to activated NK cell, NKT cell or T cell, or nucleic acid probes capable of detecting transcription products encoding the substances or plural primers (e.g., primer pair(s)) capable of amplifying them.

The kit of the present invention is, like the aforementioned agent of the present invention, useful, for example, as a kit for a pharmaceutical agent, or a kit for activation of immunocyte, or a kit for the preparation or identification of the cell of the present invention.

The contents disclosed in any publication cited in the present specification, including patents and patent applications, are hereby incorporated in their entireties by reference, to the extent that they have been disclosed herein. Examples

The present invention is explained in more detail in the following by referring to Examples, which are not to be construed as limitative.

### Material and Method

### (Mouse and cell line)

6- to 8-week-old pathogen-free C57BL/6(B6) mice were purchased from CLEA Japan (Tokyo), and B6 CD4^{-/-} and CD8^{-/-} female mice were purchased from Jackson Laboratory (Bar Harbor, ME). OT-I TCR gene recombinant mice, CD11c-DTR/GFP mice, and Jα18^{-/-} mice were provided by Dr. Heath (Walter and Eliza Hall Institute, Victoria, Australia), Dr. Littman (New York University, New York, NY) and Dr. Taniguchi (RIKEN), respectively. The above-mentioned mice were reared under particular pathogen-free conditions, and studies were performed according to the RIKEN guideline. B16, EL4 and EG7 cell lines were obtained from the American Type Culture Collection (Rockville, MD), and NIH3T3 cells were obtained from the RIKEN BANK. For transfection of CD1d, pMX-mCD1d-IRES-GFP containing mCD1d was transfected into B16 melanoma or NIH3T3 cells by retrovirus as described in J Immunol. 2007;178:2853-2861. Then, based on the GFP expression, the cell was separated by an FACS Vantage Cell Sorter.

### (Cell preparation)

DC derived from bone marrow was prepared from a bone marrow precursor cell as described in J Exp Med. 1992;176:1693-1702. On day 6, α-GalCer (100 ng/mL) was added to DC for 40 hr, during which 100 ng/mL of LPS was added for the last 16 hr. For loading of α-GalCer on other cells, in the presence of 500 ng/mL α-GalCer, fibroblast (NIH3T3 or CD1d^{hi}-NIH3T3) or tumor cell was cultured for 48 hr. The cells loaded with α-GalCer were washed 3 times before injection. CD1d^{hi}-NIH3T3 was prepared as described in J Immunol. 2007;178:2853-2861 and the like. CD70-NIH3T3, Rae1ε-NIH3T3, Rae1γ-NIH3T3 and Mult1-NIH3T3 were prepared as follows. Mouse CD70 complementary (c)DNA, Rae1ε cDNA, Rae1γ cDNA and Mult1 cDNA were cloned to retrovirus vectors having pMX-ligand cDNA-IRES-GFP, and infected with NIH3T3. Then, the cells were sorted by GFP expression.

### (Preparation of EGFP, OVA and TRP-2 mRNA)

Respective full-length cDNAs (EGFP, OVA, TRP-2) were subcloned into pSP64 poly(A) vectors (Promega, Madison, WI) (Fig. 1). Vectors having respective cDNAs were amplified, and linearized by digestion with enzyme EcoRI (for EGFP or OVA) and PvuII (for TRP-2). After preparation of capped mRNA, Ribo m⁷G cap analogs (Ambion, Austin, TX) were incorporated using RiboMax Large scale RNA large scale RNA production systems-SP6 (Promega) into Ribo Max transcription reaction to amplify mRNA (Fig. 1).

### (Transfection of mRNA)

RNA transcribed in vitro (IVT) was transfected into various cell lines using a TransMessenger transfection kit (Qiagen) according to the protocol of the manufacturer. One day before transfection, cells (2x10⁵) were seeded in a 60 mm tissue culture petri dish. The next day, the cells were washed 3 times with PBS, and transfected with a different amount of IVT RNA. The ratio of mRNA, enhancer solution, and transmessenger reagent was 1: 2: 4. The cells were transfected at different times, and directly harvested (2 hr, 4 hr, 8 hr or 16 hr), or replenished with RPMI 1640 containing 10% bovine serum albumin and cultured overnight (2 hr+16 hr culture, 4 hr+16 hr culture, 8 hr+16 hr culture or 16 hr+16 hr culture). These cells were analyzed by FACS or confocal laser scanning microscope (TCS-SP2 Leica DMRE, Heidelberg, Germany), or subjected to a measurement by ELISA (Morinaga).

### (Real time PCR assay)

Using RNeasy kit (Qiagen, Valencia, CA) or Trizol reagent (Invitrogen, Carlsbad, CA), total RNAs were isolated from various cell lines according to the protocol of manufacturer. For isolation of total RNA from a small number of cells (less than 2×10⁵) with a Trizol reagent, 5 µg of glycogen (Roche, Indianapolis, IN) was used for co-precipitation. After synthesis of cDNA from 1 µg of total RNA, mRNA expression was quantified by real-time PCR using Taqman probe primer (Applied, Biosystems).

### (In vitro tumor studies)

NIH3T3 fibroblast (5×10⁵ cells/mouse) loaded with α-GalCer and transfected with an mRNA encoding antigen was intravenously injected to the mice to immunize them. In an experiment to evaluate the development of protective immunity against tumor administration, tumor cell was subcutaneously administered to immunized mouse 2 weeks later, and the tumor size was measured. In some experiments, CD4^{-/-} and CD8^{-/-}mice were used as recipient mice.

### (Statistical analysis)

Differences in the vitro data were analyzed by Mann-Whitney U-test wherein P<0.05 was considered statistically significant.

### [Example 1]

### (Determination of optimal conditions for transfection of mRNA encoding antigen into allogeneic cells) (Fig. 2)

Optimal transfection conditions for transfection of mRNA by a chemical method using lipofection were determined. To determine the concentration-dependent transfection rate of an mRNA encoding an antigen into the cells, expression of EGFP mRNA, which was transcribed in vitro from linearized SP6 vector having EGFP, was evaluated. According to the mRNA concentration levels, EGFP expression in transfected B16 melanoma cell (H2-K^{b}) or NIH3T3 fibroblast (H2-K^{q}) was analyzed by fluorescence microscopy (Fig. 2A, B). By comparison of transfection at different mRNA concentrations, 5 µg of EGFP mRNA was determined to be sufficient for the expression of EGFP in B16 cells (Fig. 2A) and NIH3T3 cells (Fig. 2B). EGFP was sufficiently expressed in the both cells at hour 4, which continued at least for 12 hr (data not shown). As a result of FACS analysis, transfection efficiency of EGFP mRNA into B16 melanoma cell or NIH3T3 fibroblast was almost the same (Fig. 2C; data is of representative example of 3 independent experiments). It was far superior to the transfection efficiency (less than 5%; data not shown) into EL4 thymoma cell (H2-K^{b}) .

Then, the time when the protein production reaches maximum after mRNA transfection was determined. The OVA protein level produced by B16, EL4 or NIH3T3 cell transfected with 5 µg of OVA mRNA (indicated as B16-ova, EL4-ova and NIH3T3-ova, respectively) was measured by ELISA after cell lysis. By evaluation of transfection time (2-16 hr), B16-ova and NIH3T3-ova were found to produce the highest level of OVA protein at 4 hr after transfection.

Whether the cell continues to produce OVA protein after transfection was analyzed by the measurement of OVA protein level. As shown in Fig. 2D and E, expression of OVA protein by NIH3T3 transfected with OVA mRNA was of the same level as B16 transfectant; however, it continued for a longer time than B16. EL4 cell line transfected with OVA mRNA showed a low transfection level and scarcely expressed OVA protein (Fig. 2F). Thus, NIH3T3 fibroblast was selected for the subsequent experiments.

### [Example 2]

### (Transfection of CD1d gene into cell line without co-stimulatory molecule)

A tumor cell that expressed CD1d molecule can present α-GalCer on primary iNKT cells even if it does not have a costimuratory molecule. The present inventors confirmed that NIH3T3 fibroblast and B16 melanoma cell do not express CD40, CD70, CD86 and MHC class II (data not shown). As for CD1d expression, as shown in Fig. 3A and B, they analyzed parental cell lines (NIH3T3 (NIH in the Figure) and B16) and also established a stable transformant transfected with retrovirus expressing high level mouse CD1d. Stable CD1d^{hi} cell lines (CD1dNIH, CD1dB16, respectively, in the Figure) were selected at a purity of >98% by sorting using FACS Vantage Cell Sorter (Fig. 3B, left). The parental cells of B16 melanoma and NIH3T3 showed a lower CD1d expression level than bone marrow-derived DC (mBMDC in the Figure) (Fig. 3A). The CD1d expression levels of the cell lines and DC were compared by real-time PCR, and CD1d^{hi}-NIH3T3 cell (CD1dNIH in the Figure) was found to have the highest level. This finding was also confirmed by FACS (Fig. 3B, right).

### (Measurement of expression of MHC class I antigen peptide in cell lines transfected with mRNA)

An established cell line that expresses CD1d at a high level or a low level was transfected with OVA mRNA and, 4 hr later, an OVA expression level of the cell lysate was analyzed by ELISA. It was found that the OVA expression level of B16 parental cell and CD1d^{hi}-B16 transfectant (B16, CD1d-B16, respectively, in the Figure) was almost the same as that of NIH3T3 or CD1d^{hi}-NIH3T3 transfectant (NIH, CD1d-NIH, respectively, in the Figure) (Fig. 3C). It was confirmed that not only a cell line but also a transfectant containing EGFP-NIH3T3 (EGFP-NIH in the Figure) or CD1d^{hi}-NIH3T3 (CD1d-NIH in the Figure) could be stably transfected with mRNA.
A direct presentation activity of each transfectant as an antigen presenting cell was measured. To easily analyze OVA specific T cell response in vitro, B16 cell line highly expressing class I was established by exposure to recombinant IFN-γ for 12 hr (data not shown). The parental cell or transfected cell was co-cultured with CD8⁺T cells with recombined OVA specific TCR (OT-I cell, OT-1 in the Figure) for 48 hr, and the IFN-γ level of the supernatant was measured. Secretion of IFN-γ increased by the supernatant derived from the B16 cell transfected with OVA-mRNA (B16-ova; OVA-B16 in the Figure) However, such increase was not seen in the case of NIH3T3 transfected with OVA-mRNA (NIH3T3-ova; OVA-NIH in the Figure) even though it secreted OVA protein at the same level as in the B16 cell transtected with OVA-mRNA (Fig. 3D). This means that OVA peptide expresses in relation to MHC class I molecule as in OT-I and B16(K^{b}), but is a mismatch with NIH3T3 cell (K^{q}) .

### (In vivo cross presentation of OVA antigen in cell transfected with mRNA, based on the presence or absence of NKT cell aid)

As shown in Fig. 3D, despite the same level of OVA secretion, OT-I cell did not recognize peptide antigen on NIH3T3 which is mismatch with MHC class I. We ran a test to determine whether the observation applied also in vivo. To measure the in vivo antigen presenting ability of a fibroblast transfectant, OVA-mRNA transfectant loaded or not loaded with α-GalCer was given to mouse injected with OT-I cell. The absolute number of divided OT-I cells in the immunized mouse was analyzed 3 days later. As shown in Fig. 3E, OT-I cell of the mouse given CD1d^{hi}-NIH3T3 loaded with α-GalCer and transfected with OVA-mRNA (CD1d^{hi}-NIH3T3/Gal-ova; CD1d-NIH/Gal in the Figure) grew more than the OT-I cell of the mouse given CD1d^{hi}-NIH3T3 transfected with OVA-mRNA (CD1d^{hi}-NIH3T3-ova; CD1d-NIH in the Figure). The number of OT-I cells of the mouse given CD1d^{hi}-NIH3T3/Gal-ova was the same as that of the mouse given tumor/Gal, i.e., CD1d^{hi}-B16/Gal-ova (CD1d-B16/Gal in the Figure) (Fig. 3E; data shows a representative example of two independent experiments using 2 mice per group, and the difference of CD1d^{hi}-NIH3T3-ova vs CD1d^{hi}-NIH3T3/Gal-ova and CD1d^{hi}-B16-ova (CD1d-B16/Gal in the Figure) vs CD1d^{hi}-B16/Gal-ova was p<0.05 (significant). Thus, CD1d^{hi}-NIH3T3-ova cannot stimulate OT-I cell in vitro due to the MHC class I mismatch (Fig. 3D). However, CD1d^{hi}-NIH3T3/Gal-ova could grow OT-I cell in vivo. Since it grows in this way despite the MHC class I mismatch, cross presentation by endogeneous DC in an allogeneic host is suggested.

### [Example 3]

### (Fibroblast loaded with α-GalCer activates allogeneic NK and iNKT cells in vivo)

To examine whether allogeneic cell stimulates innate immunity system by α-GalCer in vivo, spleen cell of an immunized mouse was stained with CD3-FITC and NK1.1-APC, and the response of NK cell (CD3⁻NK1.1⁺) was analyzed by flow cytometry for expression of CD69 (stained with CD69-PE) and IFN-γ (stained with IFN-γ-PE) at 16 hr from immunization (Fig. 4A). In the mouse given CD1d^{hi}-NIH3T3/Gal (CD1dNIH/Gal in the Figure), NK cell increased CD69 expression and secreted IFN-γ. The NK cell of the mouse injected with NIH3T3 (NIH in the Figure) or CD1d^{hi}-NIH3T3 (CD1dNIH in the Figure) showed only a weak allogeneic response.

To analyze whether the parental cell (NIH3T3 or B16 cell) transfected with CD1d activates iNK cell by α-GalCer, spleen cells 2 days after immunization were suspended in the presence (Fig. 4B black) or absence (Fig. 4B white) of 100 ng/mL α-GalCer to re-stimulate the cells in IFN-γ ELISPOT assay. The number of IFN-γ producing spots of a mouse cell injected with NIH3T3/Gal (NIH/Gal in the Figure) or CD1d^{hi}-NIH3T3/Gal (CD1dNIH/Gal in the Figure) was similar to that of B16/Gal and CD1d^{hi}-B16/Gal (CD1B16/Gal in the Figure), respectively. The data show average of three mice per group. This suggests that CD1d^{hi}-NIH3T3/Gal and CD1d^{hi}-B16/Gal act as antigen presenting cells for the innate immune responses of iNKT cells and subsequent NK cell responses.

### (Antitumor effect via innate lymphocyte, caused by allogeneic fibroblast loaded with α-GalCer)

Then, the antitumor effect by allogeneic fibroblast transfected with mRNA and loaded with CD1d ligand was examined using B16 lung metastasis models. 2×10⁵ cells of B16 (control) were uniformly administered, 3 hr later, NIH3T3 or CD1d expression enhanced NIH3T3 (CD1d^{hi}-NIH3T3), or NIH3T3 loaded with α-GalCer (NIH3T3/Gal) or CD1d expression enhanced NIH3T3 (CD1d^{hi}-NIH3T3/Gal) (each 5×10⁵ cells) were intravenously administered to the mice of each group. Then, after 14 days from the administration, the lung was removed from each mouse, and the antitumor effect was evaluated (per group, n=5) .(Fig. 4C).

As a result, the model mice of the group administered with NIH3T3 loaded with α-GalCer (NIH3T3/Gal; NIH/Gal in the Figure) or CD1d expression enhanced NIH3T3 (CD1d^{hi}-NIH3T3/Gal; CD1dNIH/Gal in the Figure) showed a marked decrease of tumor as compared to other group. A similar effect was obtained in two independent experiments. (*) means that the difference between NIH3T3/Gal, CD1d^{hi}-NIH3T3/Gal and other groups, i.e., NIH3T3 (NIH in the Figure), CD1d^{hi}-NIH3T3 (CD1dNIH in the Figure) and control, is p<0.05 and significant.
From the foregoing, it is clear that allogeneic fibroblast transfected with mRNA and loaded with CD1d ligand shows an antitumor effect sufficient to prevent lung metastasis, by the activation of innate lymphocyte.

### [Example 4]

### (Important role of in vivo DC maturation in response to allogeneic fibroblast loaded with α-GalCer)

When a tumor cell loaded with α-GalCer is injected to a mouse, T cell response is known to require maturation of host DC after trapping an antigen. As shown in Fig. 4A and B, NIH3T3/Gal (NIH/Gal in the Figure) clearly activated natural lymphocytes. Therefore, we have tested to determine whether DC maturation occurs in vivo after injection of NIH3T3 (NIH in the Figure) or CD1d^{hi}-NIH3T3 (CD1dNIH in the Figure), loaded or unloaded with α-GalCer, into the mouse (Fig. 5A). At 12 hr after the injection, spleen cells were collected, and expression of DC surface markers (CD40, CD86 and CD119) was analyzed by flow cytometry. Like the changes of DC maturation, it was found that the expression of CD40 and CD86 increased and expression of CD119 decreased. As shown in Fig. 5A, increase of CD86 expression in DC (CD8α⁺ and CD8α⁻ subset) was similar to the increase of expression found in a mouse immunized with CD1d^{hi}-B16/Gal (CD1d^{hi}-B16/Gal in the Figure) or free α-GalCer. Since a recent report has documented that DC immunized by intravenous injection of free α-GalCer expresses CD70, CD70 expression was also analyzed similarly (Fig. 5B). It was found that CD70 level does not increase for 12 hr after injection of CD1d^{hi}-B16/Gal or CD1d^{hi}-NIH3T3/Gal (CD1dNIH/Gal in the Figure) but increases in 40 hr. It was found that a greater amount of CD70 was expressed in CD8a⁺DC than CD8a⁻DC in later stages. As a sign of maturation of functional DC, IL-12 secretion can be generally mentioned. IL-12 secretion was also analyzed (Fig. 5C).
DC derived from mouse at 4 hr from immunization by intravenous injection of NIH3T3/Gal or CD1d^{hi}-NIH3T3/Gal secreted high level of IL-12, but DC derived from mouse immunized with NIH3T3 cell or CD1d^{hi}-NIH3T3 did not. Since such DC maturation, modification of cell surface marker and IL-12 secretion are not found in Jα18-deficient mouse (data not shown), it was found that DC maturation essentially requires iNKT cells. These data suggest that DC starts maturation immediately after injection of allogeneic fibroblast loaded with α-GalCer. Not only α-GalCer directly loaded on fibroblast but also α-GalCer trapped by a host DC indirectly activates iNKT cells and mature DC.

### (DC in the body is essential for inducing adaptive immunity in CD1d^{hi}-NIH3T3/Gal-ova injection mouse)

In Fig. 3E, OT-I cell does not match with CD1D^{hi}-NIH3T3/Gal-ova (CD1dNIH/Gal in the Figure) in class I in immunized mice. Therefore, to determine whether DC of the host is involved in the presentation of OVA antigen to OT-I cell in vivo, in vivo CD11c+DC was removed from the host by using CD11c-diphtheria toxin receptor (DTR) recombinant (CD11c-DTR/GFP) mouse treated with diphtheria toxin (DT). In the mouse, OT-I cell scarcely grew, and the role of DC in the cross presentation of antigen in the mouse given a CD1d^{hi}-NIH3T3/Gal-ova cell was verified.

### [Example 5]

### (Strong adaptive immune response afforded by immunization of C57BL/6 mouse with CD1d^{hi}-NIH3T3/Gal-ova)

When our method producing an immune response to fibroblast transfected with mRNA is once established using a mouse injected with a gene recombinant OT-I cell, it becomes more important to achieve an immune response generated in a wild type mouse. We then ran a test to determine whether a wild-type mouse immunized with CD1d^{hi}-NIH3T3/Gal-ova acquires antigen specific T cell immunity (Fig. 6A). We ran a test to determine whether activation of iNKT cells and CD1d expression level in cells having the antigen are important for the induction of acquired immunity. To perform this study, mice were immunized with various parental cells or CD1d^{hi}-NIH3T3 cell transfected with OVA mRNA: NIH3T3-ova (NIH/OVA in the Figure), NIH3T3/Gal-ova (NIH/OVA/G in the Figure), CD1d^{hi}-NIH3T3-ova (CD1dNIH/OVA in the Figure) and CD1d^{hi}-NIH3T3/Gal-ova (CD1dNIH/OVA/G in the Figure). After 7 days, spleen cells were collected and the number of CD8⁺T cells specific to OVA peptide SIINFEKL (SEQ ID NO: 1) was analyzed by staining with K^{b}OVA₂₅₇-₂₆₄ tetramer. As shown in Fig. 6A, the number of the cell positive to the OVA tetramer in the mouse given NIH3T3/Gal-ova or CD1d^{hi}-NIH3T3/Gal-ova was far higher than that of the same cell in the mouse given NIH3T3-ova or CD1d^{hi}-NIH3T3-ova. However, this did not occur in a Jα-18-deficient mouse (Fig. 6B).

Then, we compared the level of T cell response after priming with iNKT cell ligand, α-GalCer, with that after priming with an NK cell ligand such as retinoic acid early inducible-1ε (Raelε), Raely, CD70, mouse UL16-binding protein-like transcript 1 (Mult1) and the like (Fig. 6C). NK cell ligand was cloned by using a retrovirus vector having EGFP. The co-expression of each molecule and EGFP was confirmed by FACS analysis (data not shown). T cell growth was evaluated by tetramer staining 1 week after immunization with CD70-NIH3T3-ova (CD70-NIH/OVA in the Figure), Rae1ε-NIH3T3-ova (Rae1ε-NIH/OVA in the Figure), Mult1-NIH3T3-ova (Mult1-NIH/OVA in the Figure) or Raelγ-NIH3T3-ova (Rae1γ-NIH/OVA in the Figure). As shown in Fig. 6C, the group immunized with Rae1ε-NIH3T3-ova or CD70-NIH3T3-ova showed a K^{b}OVA₂₅₇₋₂₆₄ tetramer positive cell proliferation, but the group immunized with other NK ligand did not. T cell response specific to OVA was also tested by using IFN-γ ELISPOT. The T cell response producing IFN-γ in mice given CD1d^{hi}-NTH3T3/Gal-ova was far higher than that in the mice given Raelγ-NIH3T3-ova, Rae1ε-NIH3T3-ova, Mult1-NIH3T3-ova, CD70-NIH3T3-ova or CD1d^{hi}-NIH3T3-ova (Fig. 6D). Thus, a fibroblast having the antigen and loaded with α-GalCer provides a stronger immune response by combining innate immunity and acquired immunity in naive mouse.

### [Example 6]

### (Induction of antitumor T cell activity by inoculation of CD1d^{hi}-NIH3T3/Gal-ova vaccine)

Whether T cell response in a mouse immunized with CD1d^{hi}-NIH3T3/Gal-ova can lead to the antitumor immunity was evaluated (Fig. 7A). A mouse was immunized by intravenous administration of 5×10⁵ cell of CD1d^{hi}-NIH3T3-ova (CD1dNIH (OVA) in the Figure) or CD1d^{hi}-NIH3T3/Gal-ova (CD1dNIH(OVA)/Gal in the Figure) and, 2 weeks later, 1×10⁵ EL4 thymoma or OVA-expressing EL4 (EG7) was administered. The mouse administered with CD1d^{hi}-NIH3T3/Gal-ova showed an antitumor effect on EG7 but not on EL4. This shows that the effect is a tumor specific immune response. The mouse given CD1d^{hi}-NIH3T3-ova (Fig. 7A) or CD1d^{hi}-NIH3T3/Gal (data not shown) developed EL4 and EG7 tumors. The defense against tumor development after intravenous inoculation requires CD4⁺ and CD8⁺T cell responses (Fig. 7B). The tumor size was measured when it was shown on the graph (per group, n=6 - 8). Similar results were obtained in two independent experiments. Whether CD1d^{hi}-NIH3T3/Gal-ova cell similarly provides defense to tumor development after 30Gy irradiation was also tested, and similar results were also found in the group of mice subjected to irradiation (data not shown).

### [Example 7]

### (Antitumor effect driven by adaptive immunity in response to NIH3T3 cell loaded with α-GalCer and transfected with trp2mRNA)

We immunized OVA model with an allogeneic cell line loaded with α-GalCer and transfected with mRNA, whereby the relationship between innate and acquired immunities was established (Fig. 7).
Then, we applied the concept to a real tumor model by immunizing the mouse with CD1d^{hi}-NIH3T3/Gal cell transfected with melanocyte differentiation antigen and tyrosinase-related protein 2 (trp2) mRNA (Fig. 8A, B). The trp2 expression in NIH3T3-trp2 (trp2-NIH in the Figure) was confirmed by RT-PCR (Fig. 8A), and confirmed by real-time PCR (Fig. 8B). The results show that the expression is nearly three times that of trp2 endogenously expressed in B16 melanoma cell.
The adaptive antitumor response to injected CD1d^{hi}-NIH3T3/Gal transfected with mRNA encoding trp2 was evaluated (Fig. 8C). Mice were immunized by intravenous administration of CD1d^{hi}-NIH3T3/Gal-trp2 (CD1dNIH(trp2)/Gal in the Figure), CD1d^{hi}-NIH3T3/Gal (CD1dNIH/Gal in the Figure) or CD1d^{hi}-NIH3T3-trp2 (CD1dNIH(trp2) in the Figure). When B16 melanoma cell (5×10⁴) was administered to the mice 2 weeks later for the evaluation of antitumor defense, the mice administered with CD1d^{hi}-NIH3T3/Gal-trp2 (Fig. 8C lower left) showed inhibition of B16 tumor growth, but the mice administered with CD1d^{hi}-NIH3T3-trp2 (Fig. 8C middle left) or CD1d^{hi}-NIH3T3/Gal (Fig. 8C upper left) showed otherwise. None of the immunized mouse groups showed an antitumor immunity against EL4 thymoma cell (1×10⁵) (Fig. 8C right). The tumor size was measured at the time point when it was shown on the graph (per group, n=6 - 8). Similar results were obtained in two independent experiments.

### Industrial Applicability

The present invention does not directly use tumor cells and pathogen-infected cells but can induce T cell capable of specifically eradicating the tumor cells and pathogen-infected cells by preparing and utilizing mRNA characteristically expressed therein. Therefore, the present invention is highly useful for the establishment of a highly effective immunotherapy for diseases caused thereby. According to the present invention, moreover, the possibility of performing an order made immunotherapy for individuals is created by clarifying the antigen property of the cell to be eradicated and preparing and using an mRNA therefor, whereby the treatment targets are drastically expanded.

This application is based on a patent application No. 2008-305639 filed in Japan, the contents of which are incorporated in full herein.

[Sequence Listing]

## Claims

1. A'method of preparing a cell co-expressing a target antigen and CD1d and having an ability to activate immunity against the target antigen, comprising the following steps (a) and (b) :
(a) a step of transfecting an mRNA encoding the target antigen into a CD1d-expressing cell to give a cell co-expressing the target antigen and CD1d; and
(b) a step of treating the cell obtained in step (a) with a CD1d ligand in a culture medium.

2. The method according to claim 1, wherein the target antigen is a tumor antigen or a pathogenic antigen.

3. The method according to claim 1, wherein the ability to activate immunity is against a tumor cell, virus or virus-infected cell.

4. The method according to claim 3, wherein the tumor cell is a solid tumor cell or a tumor cell in a hematopoietic tissue.

5. A cell having an ability to activate immunity against a target antigen, which is obtained by the method of any according to claims 1 to 4.

6. The cell according to claim 5, wherein the target antigen is a tumor antigen or pathogenic antigen.

7. The cell according to claim 5, wherein the ability to activate immunity is against a tumor cell, virus or virus-infected cell.

8. An immunoinducer to a target antigen, comprising the cell according to claim 5.

9. A composition comprising the cell according to claim 5 and an adjuvant.

10. A pharmaceutical agent comprising the cell according to claim 5.

11. The pharmaceutical agent according to claim 10, which is a therapeutic agent for a solid tumor, a tumor in a hematopoietic tissue or an infection.

12. A cell co-expressing a target antigen and CD1d, which is treated to co-express a target antigen and CD1d, or enhance expression of a target antigen and/or CD1d, and is one kind selected from the group consisting of the following (a) to (c) :
(a) a cell naturally expressing CD1d, which is transfected with an mRNA encoding the target antigen;
(b) a cell transformed with a vector expressing CD1d and transfected with an mRNA encoding the target antigen; and
(c) a cell transfected with an mRNA encoding CD1d and transfected with an mRNA encoding the target antigen.

13. A cell co-expressing a target antigen and CD1d, which is treated to co-express a target antigen and CD1d, or enhance expression of a target antigen and/or CD1d, and is one kind selected from the group consisting of the following (a) to (c):
(a) an antigen presenting cell which expresses CD1d, and is transfected with an mRNA encoding the target antigen;
(b) an antigen presenting cell which expresses CD1d, and is transformed with a vector expressing CD1d and transfected with an mRNA encoding the target antigen; and
(c) an antigen presenting cell which expresses CD1d, and is transfected with an mRNA encoding CD1d and transfected with an mRNA encoding the target antigen.

14. A kit comprising any of (1) to (8) below:
(1) a combination of (1-1) a CD1d-expressing cell, and (1-2) a construct for in vitro transcription of an mRNA encoding the target antigen;
(2) a combination of (2-1) a CD1d-expressing cell, and (2-2) an mRNA encoding the target antigen;
(3) a combination of (3-1) a construct for in vitro transcription of an mRNA encoding CD1d, (3-2) a construct for in vitro transcription of an mRNA encoding the target antigen, and (3-3) an object cell;
(4) a combination of (4-1) a construct for in vitro transcription of an mRNA encoding CD1d and an mRNA encoding the target antigen, and (4-2) an object cell;
(5) a combination of (5-1) a construct for in vitro transcription of an mRNA encoding CD1d, (5-2) an mRNA encoding the target antigen, and (5-3) an object cell;
(6) a combination of (6-1) an mRNA encoding CD1d and the target antigen, (6-2) a construct for in vitro transcription of an mRNA encoding the target antigen, and (6-3) an object cell;
(7) a combination of (7-1) an mRNA encoding CD1d and the target antigen, (7-2) an mRNA encoding the target antigen, and (7-3) an object cell; and
(8) a combination of (8-1) an mRNA encoding CD1d and the target antigen, and (8-2) an object cell.

15. The kit according to claim 14, further comprising a CD1d ligand.

16. The kit according to claim 15, which is used for induction of immunity.

17. The kit according to claim 16, wherein the CD1d-expressing cell or the object cell is allogeneic to a target in need of immunity induction.

18. The kit according to claim 17, wherein the CD1d-expressing cell or the object cell is a fibroblast.

19. A method of inducing immunity, comprising administering an effective amount of the cell according to claim 5 to a test subject (excluding human) in need thereof, wherein the cell is allogeneic to the test subject.

20. The method according to claim 19, wherein the cell is a fibroblast.

21. The method according to claim 19, further comprising administering an adjuvant.

22. Use of the cell according to claim 5, for the preparation of an immunoinducer.

23. Use according to claim 22, further comprising use of an adjuvant.
